# EUROPEAN PATENT APPLICATION

(11) **EP 3 757 218 A1**
(43) Date of publication of application: **30.12.2020**
(21) Application number: 19757738.0
(22) Date of filing: 21.02.2019
(51) Int. Cl.: C12N 15/62, C07K 14/47, C07K 19/00, C12N 1/15, C12N 1/19, C12N 1/21, C12N 5/10, C12N 15/63

(54) **FUSION PROTEIN**

(30) Priority: 21.02.2018 JP 2018029065
(71) Applicant: AJINOMOTO CO., INC., Chuo-ku Tokyo 104-8315 (JP)
(72) Inventor: INOUE, Ippei, Kawasaki-shi, Kanagawa 210-8681 (JP)
(74) Representative: Mewburn Ellis LLP
(86) International application number: PCT/JP2019/006473
(87) International publication number: WO 2019/163871

(57) **Abstract**

The present invention provides a means promising for applications such as new drug delivery systems (DDS) and preparation of electronic devices. More specifically, the present invention provides a fusion protein comprising (a) a ferritin monomer, and (b) a functional peptide inserted at a flexible linker region between α-helices in a B region and a C region of the ferritin monomer. The present invention also provides a multimer comprising a fusion protein and has an internal cavity, the fusion protein including (a) a ferritin monomer and (b) a functional peptide inserted at a flexible linker region between α-helices in a B region and a C region of the ferritin monomer.

## Description

### TECHNICAL FIELD

The present invention relates to fusion proteins and the like.

### BACKGROUND ART

Ferritin is a spherical protein that is ubiquitously present in organisms from animals and plants to microorganism and has an internal cavity formed by a plurality of monomers. In animals such as humans, it is known that two kinds of monomers of H and L chains are present as ferritin, and that ferritin is a multimer that comprises 24 monomers (in many cases, a mixture of H chain monomers and L chain monomers). Meanwhile, in microorganisms, ferritin is referred also to as Dps (DNA-binding protein from starved cells), and known to be a multimer comprising 12 monomers. It is known that ferritin is deeply involved in homeostasis of an iron element in the living organisms and cells, and holds iron inside the internal cavity thereof for playing physiological functions such as transporting and storing iron. It was shown that ferritin is able to artificially store nanoparticles including oxides of metals such as beryllium, gallium, manganese, phosphorus, uranium, lead, cobalt, nickel, and chromium, and semiconductors/magnetic substances such as cadmium selenide, zinc sulfide, iron sulfide and cadmium sulfide, in addition to iron. Thus, ferritin is actively studied on its applications in semiconductor material engineering and medical fields (Non-patent Literature 1)

Up to now, there are some reports regarding a fusion protein formed of a ferritin monomer and a peptide such as (1) a fusion protein in which a peptide is added to a terminus region of the ferritin monomer, and (2) a fusion protein in which a peptide is inserted into an internal region (region other than the terminus region) of the ferritin monomer.

For example, the following reports have been provided as the fusion protein (1).

Patent Literature 1 and Non Patent Literature 1 disclose a feature of preparing the fusion protein with titanium oxide added in one terminus region of the ferritin monomer as well as usefulness of the prepared fusion protein for preparation of electronic devices (e.g., semiconductor).

Patent literature 2 discloses a feature of preparing the fusion protein with a certain peptide added to both terminus regions of Dps as well as usefulness of the prepared fusion protein for preparation of electronic devices with special porous structures.

Regarding the fusion protein of (2), there are reports of the fusion protein with a certain peptide inserted at a flexible linker region between α-helices in D and E regions of human ferritin L chain (a region between fifth and sixth α-helices counted from the N terminus of the ferritin monomer).

For example, Non Patent Literatures 2 and 3 and Patent Literature 3 disclose a feature of preparing a fusion protein multimer (e.g., AP1-PBNC) by inserting a certain peptide (e.g., interleukin-4 receptor (IL-4R) target peptide) at a flexible linker region between α-helices in D and E regions of human ferritin L chain as well as usefulness of the multimer for treatment of diseases such as cancer.

Non Patent Literature 4 discloses a feature of preparing a fusion protein multimer by inserting a protease cleavage peptide at a flexible linker region between α-helices in D and E regions of human ferritin L chain as well as usefulness of the multimer as a protease responsive delivery system.

### PRIOR ART REFERENCES

### PATENT LITERARURES

Patent Literature 1: WO2006/126595
Patent Literature 2: WO2012/086647
Patent Literature 3: US patent application publication No. 2016/0060307

### NON PATENT LITERATURES

Non Patent Literature 1: K. Sano et al., Nano Lett., 2007, Vol. 7. p. 3200.
Non Patent Literature 2: Jae Og Jeon et al., ACS Nano (2013), 7 (9), 7462-7471.
Non Patent Literature 3: Sooji Kim et al., Biomacromolecules (2016), 17 (3), 1150-1159.
Non Patent Literature 4: Young Ji Kang et al., Biomacromolecules (2012), 13 (12), 4057-4064.

### SUMMARY OF INVENTION

### PROBLEM TO BE SOLVED BY THE INVENTION

The present invention is aimed at providing promising means for applications such as new drug delivery system (DDS), preparations of electronic devices and the like.

### SOLUTION TO PROBLEM

As a result of an extensive study, the inventors of the present application have found that a multimer comprising a fusion protein with a functional peptide being inserted at a flexible linker region between α-helices in B and C regions highly conserved in ferritin monomers of various organisms, can strongly interact with a target. For example, such a multimer can interact more strongly with a target, compared to a multimer comprising a fusion protein with a functional peptide being inserted at D or a subsequent region (e.g., a flexible linker region between α-helices in D and E regions reported in the prior arts) that is highly conserved in ferritin monomers of various organisms. Therefore, the inventors have found that such a multimer is promising for applications such as new drug delivery systems (DDS), preparations of electronic devices and the like, and completed the present invention.

That is, the present invention is as follows.
[1] A fusion protein comprising (a) a ferritin monomer, and (b) a functional peptide inserted at a flexible linker region between α-helices in a B region and a C region of the ferritin monomer.
[2] The fusion protein according to [1], wherein the ferritin monomer is a human ferritin monomer.
[3] The fusion protein according to [1] or [2], wherein the human ferritin monomer is a human ferritin H chain.
[4] The fusion protein according to [1] or [2], wherein the human ferritin monomer is a human ferritin L chain.
[5] The fusion protein according to [1], wherein the ferritin monomer is a Dps monomer.
[6] The fusion protein according to any of [1] to [5], wherein the functional peptide is a peptide capable of binding to a target material.
[7] The fusion protein according to [6], wherein the target material is an inorganic substance.
[8] The fusion protein according to [7], wherein the inorganic substance is a metal material.
[9] The fusion protein according to [6], wherein the target material is an organic substance.
[10] The fusion protein according to [9], wherein the organic substance is a biological organic molecule.
[11] The fusion protein according to [10], wherein the biological organic molecule is a protein.
[12] The fusion protein according to any of [1] to [11], wherein a cysteine residue or a peptide containing the cysteine residue is added to a C-terminus of the fusion protein.
[13] A multimer comprising a fusion protein and has an internal cavity, the fusion protein comprising (a) a ferritin monomer, and (b) a functional peptide inserted at a flexible linker region between α-helices in a B region and a C region of the ferritin monomer.
[14] A complex comprising (1) the multimer according to [13], and (2) a target material,
   wherein the target material is bound to the functional peptide in the fusion protein.
[15] A polynucleotide encoding the fusion protein according to any of [1] to [12].
[16] An expression vector comprising the polynucleotide according to [15].
[17] A host cell comprising the polynucleotide according to [15] .

### EFFECT OF THE INVENTION

A multimer comprising a fusion protein of a ferritin monomer and a functional peptide with the functional peptide being inserted at a flexible linker region between α-helices in B and C regions in the ferritin monomer can strongly interact with a target. The present invention provides not only such a multimer with the superior interaction capability but also the fusion protein that is a monomer to be used for preparation of such a multimer, as well as a complex formed of such a multimer. The present invention also provides polynucleotides, expression vectors and host cells useful for preparation of such fusion proteins, multimers and complexs.

### Brief Description of Drawings

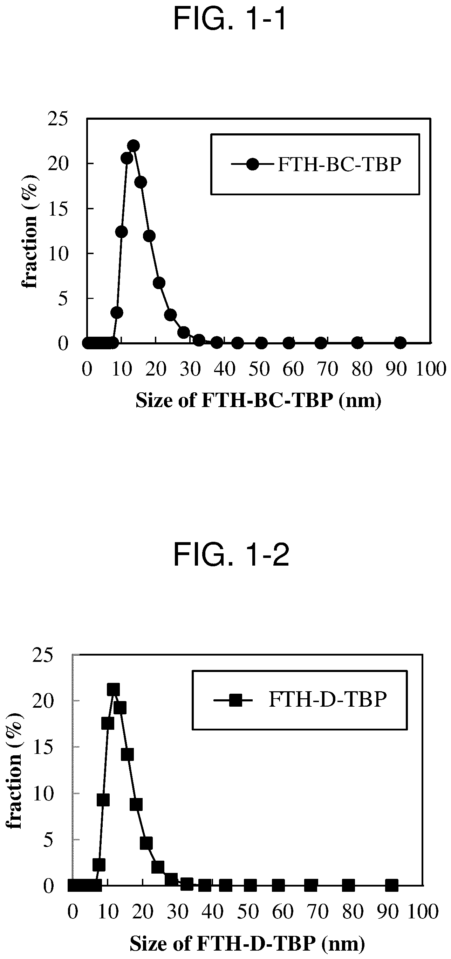

FIG. 1-1 is a graph representing an evaluation of solution dispersibility for different particle sizes of FTH-BC-TBP using dynamic light scattering (DLS). The FTH-BC-TBP refers to a human-derived ferritin H chain with a titanium recognizing peptide (minTBP1) being inserted for fusion at a flexible linker region between second and third α-helices counted from N-terminus of a ferritin monomer comprising six α-helices.
FIG. 1-2 is a graph representing an evaluation of solution dispersibility for different particle sizes of FTH-D-TBP using dynamic light scattering (DLS). The FTH-D-TBP refers to a human-derived ferritin H chain with a gold recognizing peptide (GBP1) being inserted for fusion at a flexible linker region between fourth and fifth α-helices counted from N-terminus of the ferritin monomer comprising six α-helices.

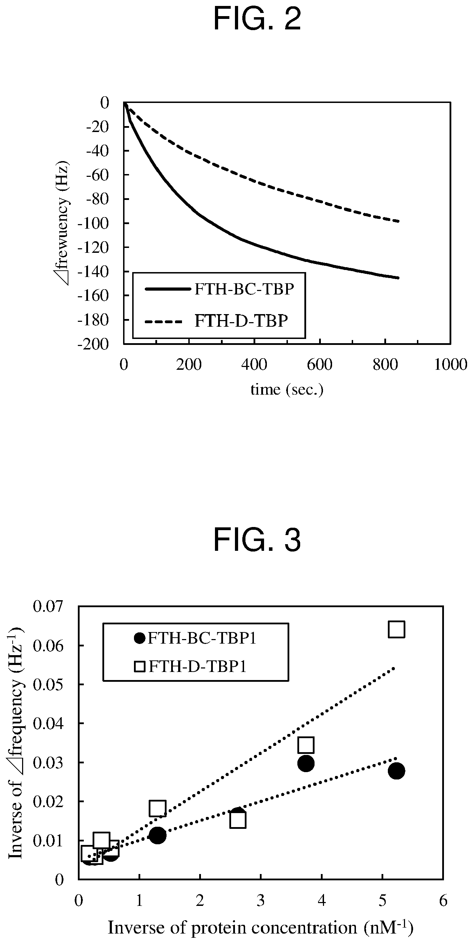

FIG. 2 is a graph representing an evaluation of adsorbabilities of FTH-BC-TBP and FTH-D-TBP to a titanium film using quartz crystal microbalance (QCM) method.
FIG. 3 is a graph representing frequency changes with different concentrations of FTH-BC-TBP and FTH-D-TBP measured by quartz crystal microbalance (QCM) method. After the measurement of frequency changes with the different concentrations, dissociation equilibrium constant KD values are determined from slopes obtained by plots for correlations between reciprocals of the different concentrations and reciprocals of the frequency changes.

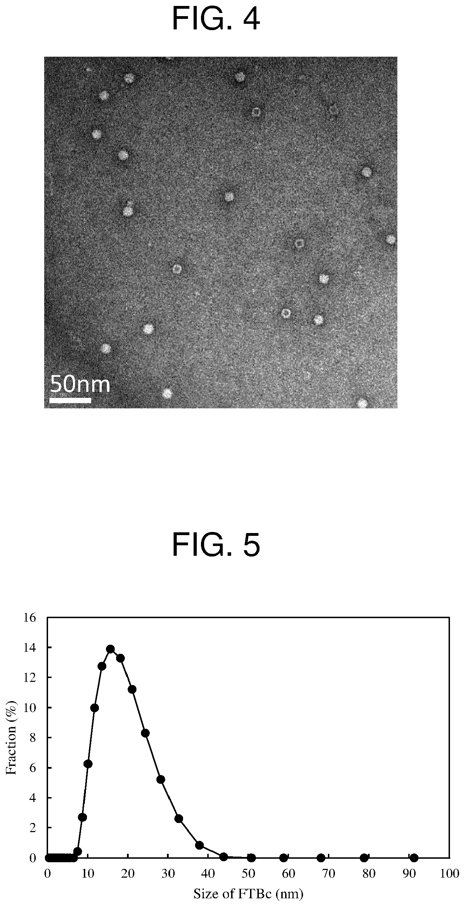

FIG. 4 is a transmission electron microscopic (TEM) image (cage-like shape) with a 3% phosphotungstic acid staining for FHBc. The FHBc is a human-derived ferritin H chain with a cancer recognizing RGD peptide being inserted for fusion at a flexible linker region between the second and third α-helices counted from N-terminus of the ferritin monomer comprising six α-helices.
FIG. 5 is a graph representing an evaluation of solution dispersibility for different particle sizes of FHBc encapsulated with iron oxide nanoparticles using dynamic light scattering (DLS) method.

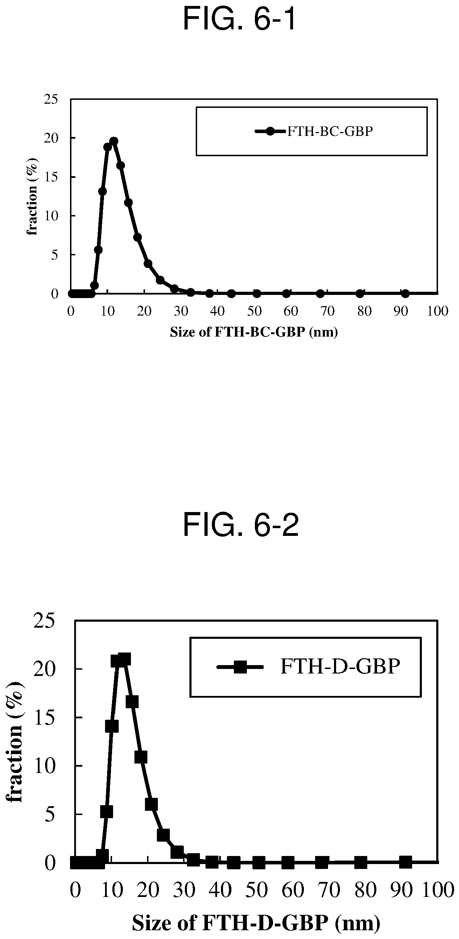

FIG. 6-1 is a graph representing an evaluation of solution dispersibility for different particle sizes of FTH-BC-GBP using dynamic light scattering (DLS). The FTH-BC-GBP refers to a human-derived ferritin H chain with a gold recognizing peptide (GBP 1) being inserted for fusion at a flexible linker region between second and third α-helices counted from N-terminus of the ferritin monomer comprising six α-helices.
FIG. 6-2 is a graph representing an evaluation of solution dispersibility for different particle sizes of FTH-D-GBP using dynamic light scattering (DLS). The FTH-D-GBP refers to a human-derived ferritin H chain with a gold recognizing peptide (GBP1) being inserted for fusion at a flexible linker region between fourth and fifth α-helices counted from N-terminus of the ferritin monomer comprising six α-helices.

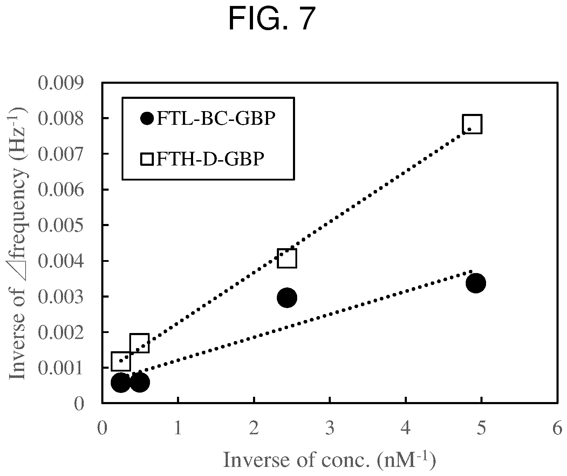

FIG. 7 is a graph representing an evaluation of adsorbabilities of FTH-BC-GBP and FTH-D-GBP to a gold film using quartz crystal microbalance (QCM) method. After the measurement of frequency changes with different concentrations, dissociation equilibrium constant KD values are determined from slopes obtained by plots for correlations between reciprocals of the different concentrations and reciprocals of the frequency changes.

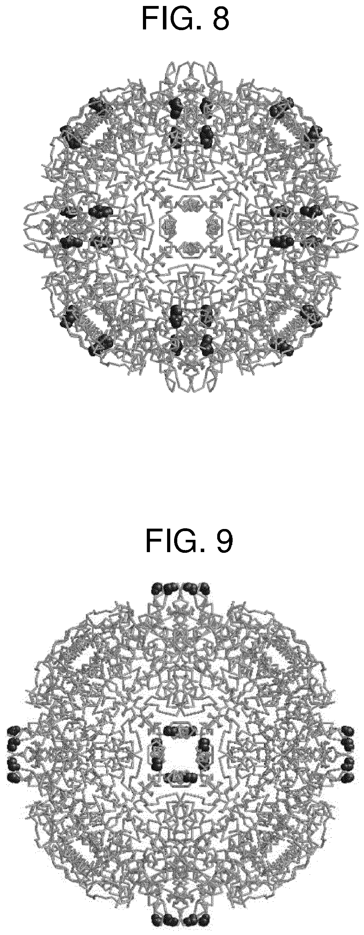

FIG. 8 shows a schematic three-dimensional structure of FTL-BC-GBP. The FTL-BC-GBP refers to a human-derived ferritin L chain with a gold recognizing peptide (GBP1) being inserted for fusion at a flexible linker region between second and third α-helices counted from N-terminus of the ferritin monomer comprising six α-helices.
FIG. 9 shows a schematic three-dimensional structure of FTL-DE-GBP. The FTL-DE-GBP refers to a human-derived ferritin L chain with a gold recognizing peptide (GBP1) being inserted for fusion at a flexible linker region between fifth and sixth α-helices counted from N-terminus of the ferritin monomer comprising six α-helices.

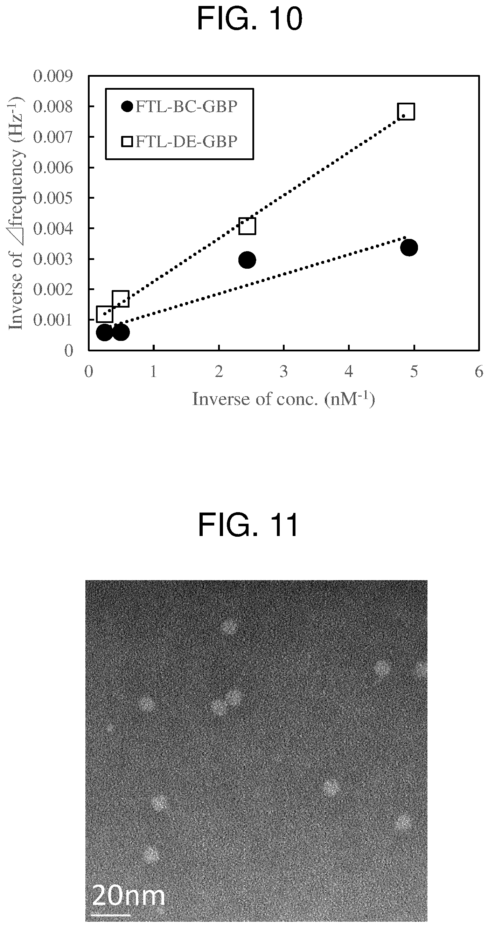

FIG. 10 is a graph representing an evaluation of adsorbabilities of FTL-BC-GBP and FTL-DE-GBP to a gold film using quartz crystal microbalance (QCM) method. After measurement of frequency changes with the different concentrations of FTL-BC-GBP and FTL-DE-GBP, dissociation equilibrium constant KD values are determined from slopes obtained by plots for correlations between reciprocals of the different concentrations and reciprocals of the frequency changes.
FIG. 11 is a transmission electron microscopic (TEM) image (cage-like shape) with a 3% phosphotungstic acid staining for BCDps-CS4. The BCDps-CS4 refers to a Listeria innocua-derived Dps with a heterologous peptide being inserted at a region corresponding to ferritin and C-terminus.

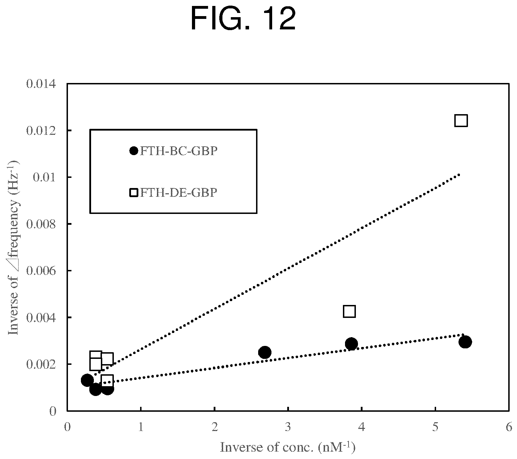

FIG. 12 is a graph representing an evaluation of adsorbabilities of FTH-BC-GBP and FTH-DE-GBP to a gold film using quartz crystal microbalance (QCM) method. After measurement of frequency changes with the different concentrations of FTH-BC-GBP and FTH-DE-GBP, dissociation equilibrium constant KD values are determined from slopes obtained by plots for correlations between reciprocals of the different concentrations and reciprocals of the frequency changes.

### EMBODIMENTS FOR CARRYING OUT THE INVENTION

The present invention provides a fusion protein comprising (a) a ferritin monomer, and (b) a functional peptide inserted at a flexible linker region between α-helices in B and C regions of the ferritin monomer.

Ferritin (multimeric protein) is universally present in various organisms. In the present invention, ferritin monomers of various organisms can be used as the ferritin monomer constituting ferritin. Examples of the organism from which the ferritin monomer is derived include higher organisms such as animals, insects, fishes and plants, as well as microorganisms. As the animals, mammals or avian species (e.g., chicken) are preferable, and mammals are more preferable. Examples of mammals include primates (e.g., humans, monkeys, chimpanzees), rodents (e.g., mice, rats, hamsters, guinea pigs, rabbits), and livestock and working mammals (e.g., cattle, pigs, sheep, goats and horses). Either H chain or L chain can be used as the ferritin monomer. Either naturally occurring ferritin monomer or a mutant thereof can be used as the ferritin monomer.

In one embodiment, the ferritin monomer is a human ferritin monomer. From the viewpoint of clinical application to humans, it is preferable to use the human ferritin monomer as the ferritin monomer. As the human-derived ferritin monomer, either human ferritin H chain or human ferritin L chain can be used.

Preferably, the human ferritin H chain may be as follows:
(A1) a protein comprising the amino acid sequence of SEQ ID NO: 2;
(B1) a protein comprising an amino acid sequence including one or several modification(s) of an amino acid residue(s) selected from the group consisting of substitution, deletion, insertion and addition of the amino acid residue(s) in the amino acid sequence of SEQ ID NO: 2, the protein capable of forming a multimer (e.g., 24-mer); or
(C1) a protein comprising an amino acid sequence having 90% or more homology to the amino acid sequence of SEQ ID NO: 2, the protein capable of forming a multimer (e.g., 24-mer).

Preferably, the human ferritin L chain may be as follows:
(A2) a protein comprising the amino acid sequence of SEQ ID NO: 4;
(B2) a protein comprising an amino acid sequence including one or several modification(s) of an amino acid residue(s) selected from the group consisting of substitution, deletion, insertion and addition of the amino acid residue(s) in the amino acid sequence of SEQ ID NO: 4, the protein capable of forming a multimer (e.g., 24-mer); or
(C2) a protein comprising an amino acid sequence having 90% or more homology to the amino acid sequence of SEQ ID NO: 4, the protein capable of forming a multimer (e.g., 24-mer).

In another embodiment, the ferritin monomer is a microbial ferritin monomer. The microorganism ferritin is referred also to as Dps. Dps may be referred to as NapA, bacterioferritin, Dlp or MrgA depending on the kinds of bacteria derived from Dps, and has several sub-types such as DpsA, DpsB, Dps1 and Dps2 (see T. Haikarainen and A. C. Papageorgion, Cell. Mol. Life Sci., 2010 vol. 67, p. 341). In the present invention, monomers of Dps or the alternatively termed proteins can be used as the microbial ferritin monomer.

As the microbial ferritin, ferritin of various microorganisms is known (e.g., WO2012/086647). Examples of such microorganisms include bacteria belonging to Listeria, Staphylococcus, Bacillus, Streptococcus, Vibrio, Escherichia, Brucella, Borrelia, Mycobacterium, Campylobacter, Thermosynechococcus, Deinococcus and Corynebacterium. Examples of bacteria belonging to Listeria include Listeria innocua and Listeria monocytogeneses. Staphylococcus Aureus is an example of bacteria belonging to Staphylococcus. Bacillus subtilis is an example of bacteria belonging to Bacillus. Examples of bacteria belonging to Streptococcus include Streptococcus pyogenes and Streptococcus suis. Vibrio cholerae is an example of bacteria belonging to Vibrio. Escherichia coli is an example of bacteria belonging to Escherichia. Brucella Melitensis is an example of bacteria belonging to Brucella. Borrelia Burgdorferi is an example of bacteria belonging to Borrelia. Mycobacterium smegmatis is an example of bacteria belonging to Mycobacterium. Campylobacter jejuni is an example of bacteria belonging to Campylobacter. Thermosynechococcus Elongatus is an example of bacteria belonging to Thermosynechococcus. Deinococcus Radiodurans is an example of bacteria belonging to Deinococcus. Corynebacterium glutamicum is an example of bacteria belonging to Corynebacterium. In the present invention, ferritin monomers of the above microorganisms can be used as the microbial ferritin monomer.

Preferably, the microbial ferritin monomer is Listeria innocua ferritin (Dps) monomer. The Listeria innocua ferritin (Dps) monomer may be as follows:
(A3) a protein comprising the amino acid sequence of SEQ ID NO: 6;
(B3) a protein comprising an amino acid sequence including one or several modification(s) of an amino acid residue(s) selected from the group consisting of substitution, deletion, insertion and addition of the amino acid residue(s) in the amino acid sequence of SEQ ID NO: 6, the protein capable of forming a multimer (e.g., 12-mer); or
(C3) a protein comprising an amino acid sequence having 90% or more homology to the amino acid sequence of SEQ ID NO: 6, the protein capable of forming a multimer (e.g., 12-mer).

In the proteins (B1) to (B3), one or several amino acid residue(s) can be modified by one, two, three or four kinds of modifications selected from the group consisting of deletion, substitution, addition and insertion of amino acid residues. The modification of the amino acid residues may be introduced into one region in the amino acid sequence, or may be introduced into a plurality of different regions. The term "one or several" represents the number that is selected not to impair activities of the protein. The number represented by the term "one or several" refers to 1 to 50, for example, preferably 1 to 40, more preferably 1 to 30, still more preferably 1 to 20, and particularly preferably 1 to 10 or 1 to 5 (e.g., 1, 2, 3, 4 or 5).

In the proteins (C1) to (C3), the extent of homology to the amino acid sequence of interest is preferably 92% or more, more preferably 95% or more, still more preferably 97% or more, and most preferably 98% or more or 99% or more. The homology (i.e., identity or similarity) of the amino acid sequence can be determined by algorithm BLAST (Pro. Natl. Acad. Sci. USA, 90, 5873

(1993)) developed by Karlin and Altschul, and FASTA (Methods Enzymol., 183, 63 (1990)) developed by Pearson. Since programs called BLASTP or BLASTN have been developed based on the algorithm BLAST (see http://www.ncbi.nlm.nih.gov), these programs may be used in default settings to calculate the homology. For example, a numerical value can be used that is obtained by using software GENETYX Ver 7.0.9, which is developed by GENETYX Corporation and employs Lipman-Pearson method, for performing percentage calculation of the similarity as the homology using the entire length of the partial polypeptide encoded in the ORF by settings of Unit Size to Compare = 2. The homology may be a value (Identity) obtained by using default setting parameters (Gap penalty = 10, Extend penalty = 0.5, Matrix = EBLOSUM62) in searching with NEEDLE program (J Mol Biol 1970; 48: 443-453). The lowest value can be employed among the values of homology % derived by these calculations. Identity % is preferably used as the homology %.

The position of the amino acid residue to which the mutation is introduced in the amino acid sequence is apparent to a person skilled in the art, but may be determined with further reference to the sequence alignment. Specifically, a person skilled in the art can (1) compare a plurality of amino acid sequences, (2) reveal a relatively conserved region and a region that is not relatively conserved, and (3) then predict a region capable of playing important roles for functions and a region incapable of playing important roles for functions from the relatively conserved region and the region that is not relatively conserved, respectively, for recognition of correlations between structures and functions. As such, a person skilled in the art can determine the position to which the mutation should be introduced in the amino acid sequence by utilizing the sequence alignment, as well as the position of the amino acid residue to which the mutation should be introduced in the amino acid sequence by combination use of known secondary and tertiary structure information.

When the amino acid residue is mutated by substitution, the substitution of the amino acid residue may be a conservative substitution. The term "conservative substitution" used in this description refers to replacing a certain amino acid residue with an amino acid residue having an analogous side chain. Families of the amino acid residues with analogous side chains are known in the relevant field. Examples of such families include amino acids with basic side chains (e.g., lysine, arginine and histidine), amino acids with acidic side chains (e.g., aspartic acid and glutamic acid), amino acids with uncharged polar side chains (e.g., glycine, asparagine, glutamine, serine, threonine, tyrosine and cysteine), amino acids with non-polar side chains (e.g., alanine, valine, leucine, isoleucine, proline, phenylalanine, methionine and tryptophan), amino acids with β-branched amino acids (e.g., threonine, valine and isoleucine), amino acids with aromatic side chains (e.g., tyrosine, phenylalanine, tryptophan and histidine), amino acids with hydroxy group (e.g., alcoholic and phenolic)-containing side chains (e.g., serine, threonine and tyrosine), and amino acids with sulfur-containing side chains (e.g., cysteine and methionine). Preferably, the conservative substitutions of amino acids may be substitution between aspartic acid and glutamic acid, substitution among arginine, lysine and histidine, substitution between tryptophan and phenylalanine, substitution between phenylalanine and valine, substitution among leucine, isoleucine and alanine, and substitution between glycine and alanine.

It is known that the ferritin monomer of higher organisms has six α-helices highly conserved in various higher organisms, and that there are two kinds of monomers, H chain and L chain, as the ferritin monomers of higher organisms. It is known that the ferritin monomer (Dps monomer) of microorganisms has five α-helices highly conserved in various microorganisms, and that there is one kind of monomer as the ferritin monomer of the microorganism. The ferritin monomers of higher organisms and microorganisms have α-helices highly conserved in A region, B region, C region and D region. In the ferritin monomer of higher organisms, α-helix deletion is recognized in the boundary between the B region and the C region present in the ferritin monomer of microorganisms. In the ferritin monomer of microorganisms, α-helix deletion is recognized in the E region present in the ferritin monomer of higher organisms. Positions of α-helices are summarized in the following Table 1, for human ferritin monomer listed as an example of the ferritin monomer of higher organism and Listeria innocua ferritin monomer listed as an example of the ferritin monomer of microorganism.

**Table 1. α-helix position of ferritin**

| α-helix | position of amino acid residue in amino acid sequence | | |
|---|---|---|---|
| | human ferritin | | Listeria innocua |
| | H chain (SEQ ID NO: 2) | L chain (SEQ ID NO: 4) | Dps monomer (SEQ IDNO: 6) |
| A region | 15-42 (1st) | 11-37 (1st) | 9-33 (1st) |
| B region | 50-77 (2nd) | 46-73 (2nd) | 39-66 (2nd) |
| Boundary between B and C regions | none | none | 75-81 (3rd) |
| C region | 97-124 (3rd) | 93-120 (3rd) | 95-122 (4th) |
| D region | 128-137 (4th) 139-159 (5th) | 124-133 (4th) 135-154 (5th) | 126-149 (5th) |
| E region | 165-174 (6th) | 160-170 (6th) | none |

| | | | |
|---|---|---|---|
| (a) Number in parenthesis (Xth) represents Xth α-helix at Xth position counted from N-terminus. (b) Classification of A to E regions and first to sixth are defined according to Int J Mol Sci. 2011;12(8):5406-5421 | | | |

Stronger interaction with a target can be achieved in a multimer comprising a fusion protein in which a functional peptide is inserted at a flexible linker region between α-helices in the B and C regions highly conserved in the ferritin monomers of various organisms (a region between second and third α-helices counted from the N-terminus of the ferritin monomer in higher organisms such as human; and a region between second and fourth α-helices counted from the N-terminus of the ferritin (Dps) monomer in microorganisms), compared to a multimer comprising a fusion protein in which a functional peptide is inserted in D and/or subsequent region(s) highly conserved in the ferritin monomers of various organisms [for example, a flexible linker region between α-helices in D and E regions reported in the prior arts (for example, a region between fifth and sixth α-helices counted from the N-terminus of the ferritin monomer).

The α-helices in the B and C regions of the ferritin monomer are well-known in the relevant field, and a person skilled in the art can determine the positions of α-helices in the B and C regions of the ferritin monomer derived from various organisms as appropriate. Therefore, the flexible linker region between α-helices in the B and C regions in which the functional peptide is inserted in the present invention, is also well-known in the relevant field, and can be determined by a person skilled in the art as appropriate. For example, as such a position for the insertion of functional peptide in the H chain of higher organisms such as human ferritin H chain (SEQ ID NO: 2), any position can be utilized in a region including amino acid residues at positions 78 to 96 (preferably positions 83 to 91). As such a position for the insertion of functional peptide in the L chain of higher organisms such as human ferritin L chain (SEQ ID NO: 4), any position can be utilized in a region including amino acid residues at positions 74 to 92 (preferably positions 79 to 87). As such a position for the insertion of functional peptide in the ferritin monomer Dps of microorganisms such as Listeria innocua Dps (SEQ ID NO: 6), any position can be utilized in a region including amino acid residues at positions 67 to 94 (preferably positions 82 to 94). In various fusion proteins constructed in Examples, various functional peptides (for example, titanium recognizing peptide, cancer recognizing peptide and gold recognizing peptide) are inserted at certain positions in the certain ferritin monomers as listed below in Table 2.

**Table 2. Insertion position of functional peptide in various fusion proteins constructed in Examples**

| Example 1 FTH-BC-TBP | human ferritin H chain | between positions 87 and 88 |
|---|---|---|
| FTH-D-TBP | human ferritin H chain | between positions 137 and 138 |
| Example 2 FHBc | human ferritin H chain | between positions 87 and 88 |
| Example 5 FTH-BC-GBP | human ferritin H chain | between positions 87 and 88 |
| FTH-D-GBP | human ferritin H chain | between positions 137 and 138 |
| Example 7 FTL-BC-GBP | human ferritin L chain | between positions 83 and 84 |
| FTL-DE-GBP | human ferritin L chain | between positions 156 and 157 |
| Example 9 BcDps-CS4 | Listeria innocua ferritin monomer Dps | between positions 90 and 91 |
| Example 11 FTH-DE-GBP | human ferritin H chain | between positions 162 and 163 |

| | | |
|---|---|---|
| human ferritin H chain: SEQ ID NO: 2 human ferritin L chain: SEQ ID NO: 4 Listeria innocua ferritin monomer Dps: SEQ ID NO: 6 | | |

As the functional peptide, a peptide that enables addition of any function to a target protein can be used when fused with the target protein. Examples of such a peptide include a peptide capable of binding to a target material, a protease degrading peptide, a cell-permeable peptide and a stabilizing peptide. The present invention reveals that superior capability of binding to the target material can be achieved by a multimer comprising a fusion protein in which the peptide capable of binding to the target material is inserted at the region between second and third α-helices of ferritin, compared to a multimer comprising a fusion protein in which the same peptide is inserted at a region between fifth and sixth α-helices of ferritin. This indicates that the peptide inserted in the region between the second and third α-helices can interact more strongly with the target than the peptide inserted in the region between the fifth and sixth α-helices.As such, the strong interaction with the target (e.g., protease) can be expected in the case of using not only the peptide capable of binding to the target material but also another functional peptide (e.g., protease-degrading peptide). Therefore, the present invention is useful also when another protein is used as the functional peptide.

The functional peptide to be inserted in the above region may refer to one peptide with a desired function, or same type or different types of plural (e.g., several such as two, three, four) peptide with desired functions. When the functional peptide refers to the peptides as described above, the functional peptides can be inserted in any order and fused with the ferritin monomer. The fusion can be achieved through an amide bond(s). The fusion may be achieved directly by the amide bond(s), or indirectly by the amide bond(s) mediated by one amino acid residue (e.g., methionine) or a peptide (peptide linker) comprising several (e.g., 2 to 20, preferably 2 to 10, more preferably 2, 3, 4 or 5) amino acid residues. Since various peptide linkers are known, such a peptide linker can be used in the present invention. Preferably, the total length of the peptide to be inserted in the above-mentioned region is 20 amino acid residues or less.

When the peptide capable of binding to the target material is used as the functional peptide, examples of the target material include an organic substance and an inorganic substance (e.g., conductor, semiconductor and magnetic substance). More specifically, examples of such a target material include biological organic molecules, metal materials, silicon materials, carbon materials, materials (e.g., nickel, maltose and glutathione) capable of interacting with a tag for protein purification (e.g., histidine tags, maltose-binding protein tags, glutathione-S-transferase), labeling substances (e.g., radioactive substances, fluorescent substances and dyes), polymers (e.g., hydrophobic organic polymers or conductive polymers such as polymethylmethacrylate, polystyrene, polyethylene oxide and poly(L-lactic acid)).

Examples of biological organic molecules include proteins (e.g., oligopeptide or polypeptide), nucleic acids (e.g., DNA, RNA, nucleosides, nucleotides, oligonucleotides or polynucleotides), saccharides (e.g., monosaccharides, oligosaccharides or polysaccharides) and lipids. The biological organic molecule may also be a cell surface antigen (e.g., a cancer antigen, a heart disease marker, a diabetes marker, a neurological disease marker, an immune disease marker, an inflammatory marker, a hormone, an infectious disease marker). The biological organic molecule may also be a disease antigen (e.g., a cancer antigen, a heart disease marker, a diabetes marker, a neurological disease marker, an immune disease marker, an inflammatory marker, a hormone, an infectious disease marker). Various peptides have been reported as peptides capable of binding to such biological organic molecules. Several peptides have been reported as follows: for example, peptides capable of binding to protein (see, e.g., F. Danhier et al., Mol. Pharmaceutics, 2012, vol. 9, No. 11, p. 2961; C-H. Wu et al., Sci. Transl. Med., 2015, Vol. 7, No. 290, 290ra91; L. Vannucci et al., Int. J. Nanomedicine. 2012, Vol. 7, p. 1489; J. Cutrera et al., Mol. Ther. 2011, Vol 19 (8), p. 1468; R. Liu et al., Adv. Drug Deliv. Rev. 2017, Vol. 110-111, p. 13); peptides capable of binding to nucleic acid (see, e.g., R. Tan et al., Proc. Natl. Acad. Sci. USA, 1995, vol. 92, p. 5282; R. Tan et al., Cell, 1993, vol. 73, p 1031; R. Talanian et al., Biochemistry. 1992, Vol. 31, p. 6871); peptides capable of binding to saccharide (see, e.g., K. Oldenburg et al., Proc. Natl. Acad. Sci. USA, 1992, vol. 89, No. 12, p. 5393-5397; K. Yamamoto et al., J. Biochem., 1992, vol. 111, p. 436; A. Baimiev et al., Mol. Biol. (Moscow), 2005, vol. 39, No. 1, p. 90); and peptides capable of binding to lipid (see, e.g., O. Kruse et al., B Z. Naturforsch., 1995, Vol. 50c, p. 380; O. Silva et al., Sci. Rep., 2016, Vol. 6, 27128; A. Filoteo et al., J. Biol. Chem., 1992, vol. 267, No. 17, p. 11800).

Preferably, the peptide capable of binding to biological organic molecule may be the peptide capable of binding to protein. Examples of the peptide capable of binding to protein include RGD-containing peptides disclosed in Danhier et al., Mol. Pharmaceutics, 2012, vol. 9, No. 11, p. 2961, and modified sequence thereof (e.g., RGD (SEQ ID NO: 37), ACDCRGDCFCG (SEQ ID NO: 38), CDCRGDCFC (SEQ ID NO: 39), GRGDS (SEQ ID NO: 40), ASDRGDFSG (SEQ ID NO: 16)), and other integrin recognition sequences (e.g., EILDV (SEQ ID NO: 41) and REDV (SEQ ID NO: 42)), peptides disclosed in L. Vannucci et al., Int. J. Nanomedicine. 2012, vol. 7, p. 1489 (e.g., SYSMEHFRWGKP (SEQ ID NO: 43)), peptides disclosed in J. Cutrera et al., Mol. Ther. 2011, vol. 19, No. 8, p. 1468 (e.g., VNTANST (SEQ ID NO: 44)), peptides disclosed in R. Liu et al., Adv. Drug Deliv. Rev. 2017, vol. 110-111, p. 13 (e.g., DHLASLWWGTEL (SEQ ID NO: 45) and NYSKPTDRQYHF (SEQ ID NO: 46), IPLPPPSRPFFK (SEQ ID NO: 47), LMNPNNHPRTPR (SEQ ID NO: 48), CHHNLTHAC (SEQ ID NO: 49), CLHHYHGSC (SEQ ID NO: 50), CHHALTHAC (SEQ ID NO: 51), SPRPRHTLRLSL (SEQ ID NO: 52), TMGFTAPRFPHY (SEQ ID NO: 53), NGYEIEWYSWVTHGMY (SEQ ID NO: 54), FRSFESCLAKSH (SEQ ID NO: 55), YHWYGYTPQNVI (SEQ ID NO: 56), QHYNIVNTQSRV (SEQ ID NO: 57), QRHKPRE (SEQ ID NO: 58), HSQAAVP (SEQ ID NO: 59), AGNWTPI (SEQ ID NO: 60), PLLQATL (SEQ ID NO: 61), LSLITRL (SEQ ID NO: 62), CRGDCL (SEQ ID NO: 63), CRRETAWAC (SEQ ID NO: 64), RTDLDSLRTYTL (SEQ ID NO: 65), CTTHWGFTLC (SEQ ID NO: 66), APSPMIW (SEQ ID NO: 67), LQNAPRS (SEQ ID NO: 68), SWTLYTPSGQSK (SEQ ID NO: 69), SWELYYPLRANL (SEQ ID NO: 70), WQPDTAHHWATL (SEQ ID NO: 71), CSDSWHYWC (SEQ ID NO: 72), WHWLPNLRHYAS (SEQ ID NO: 73), WHTEILKSYPHE (SEQ ID NO: 74), LPAFFVTNQTQD (SEQ ID NO: 75), YNTNHVPLSPKY (SEQ ID NO: 76), YSAYPDSVPMMS (SEQ ID NO: 77), TNYLFSPNGPIA (SEQ ID NO: 78), CLSYYPSYC (SEQ ID NO: 79), CVGVLPSQDAIGIC (SEQ ID NO: 80), CEWKFDPGLGQARC (SEQ ID NO: 81), CDYMTDGRAASKIC (SEQ ID NO: 82), KCCYSL (SEQ ID NO: 83), MARSGL (SEQ ID NO: 84), MARAKE (SEQ ID NO: 85), MSRTMS (SEQ ID NO: 86), WTGWCLNPEESTWGFCTGSF (SEQ ID NO: 87), MCGVCLSAQRWT (SEQ ID NO: 88), SGLWWLGVDILG (SEQ ID NO: 89), NPGTCKDKWIECLLNG (SEQ ID NO: 90), ANTPCGPYTHDCPVKR (SEQ ID NO: 91), IVWHRWYAWSPASRI (SEQ ID NO: 92), CGLIIQKNEC (SEQ ID NO: 93), MQLPLAT (SEQ ID NO: 94), CRALLRGAPFHLAEC (SEQ ID NO: 95), IELLQAR (SEQ ID NO: 96), TLTYTWS (SEQ ID NO: 97), CVAYCIEHHCWTC (SEQ ID NO: 98), THENWPA (SEQ ID NO: 99), WHPWSYLWTQQA (SEQ ID NO: 100), VLWLKNR (SEQ ID NO: 101), CTVRTSADC (SEQ ID NO: 102), AAAPLAQPHMWA (SEQ ID NO: 103), SHSLLSS (SEQ ID NO: 104), ALWPPNLHAWVP (SEQ ID NO: 105), LTVSPWY (SEQ ID NO: 106), SSMDIVLRAPLM (SEQ ID NO: 107), FPMFNHWEQWPP (SEQ ID NO: 108), SYPIPDT (SEQ ID NO: 109), HTSDQTN (SEQ ID NO: 110), CLFMRLAWC (SEQ ID NO: 111), DMPGTVLP (SEQ ID NO: 112), DWRGDSMDS (SEQ ID NO: 113), VPTDTDYS (SEQ ID NO: 114), VEEGGYIAA (SEQ ID NO: 115), VTWTPQAWFQWV (SEQ ID NO: 116), AQYLNPS (SEQ ID NO: 117), CSSRTMHHC (SEQ ID NO: 118), CPLDIDFYC (SEQ ID NO: 119), CPIEDRPMC (SEQ ID NO: 120), RGDLATLRQLAQEDGVVG (SEQ ID NO: 121), SPRGDLAVLGHK (SEQ ID NO: 122), SPRGDLAVLGHKY (SEQ ID NO: 123), CQQSNRGDRKRC (SEQ ID NO: 124), CMGNKCRSAKRP (SEQ ID NO: 125), CGEMGWVRC (SEQ ID NO: 126), GFRFGALHEYNS (SEQ ID NO: 127), CTLPHLKMC (SEQ ID NO: 128), ASGALSPSRLDT (SEQ ID NO: 129), SWDIAWPPLKVP (SEQ ID NO: 130), CTVALPGGYVRVC (SEQ ID NO: 131), ETAPLSTMLSPY (SEQ ID NO: 132), GIRLRG (SEQ ID NO: 133), CPGPEGAGC (SEQ ID NO: 134), CGRRAGGSC (SEQ ID NO: 135), CRGRRST (SEQ ID NO: 136), CNGRCVSGCAGRC (SEQ ID NO: 137), CGNKRTRGC (SEQ ID NO: 138), HVGGSSV (SEQ ID NO: 139), RGDGSSV (SEQ ID NO: 140), SWKLPPS (SEQ ID NO: 141), CRGDKRGPDC (SEQ ID NO: 142), GGKRPAR (SEQ ID NO: 143), RIGRPLR (SEQ ID NO: 144), CGFYWLRSC (SEQ ID NO: 145), RPARPAR (SEQ ID NO: 146), TLTYTWS (SEQ ID NO: 147), SSQPFWS (SEQ ID NO: 148), YRCTLNSPFFWEDMTHEC (SEQ ID NO: 149), KTLLPTP (SEQ ID NO: 150), KELCELDSLLRI (SEQ ID NO: 151), IRELYSYDDDFG (SEQ ID NO: 152), NVVRQ (SEQ ID NO: 153), VECYLIRDNLCIY (SEQ ID NO: 154), CGGRRLGGC (SEQ ID NO: 155), WFCSWYGGDTCVQ (SEQ ID NO: 156), NQQLIEEIIQILHKIFEIL (SEQ ID NO: 157), KMVIYWKAG (SEQ ID NO: 158), LNIVSVNGRH (SEQ ID NO: 159), QMARIPKRLARH (SEQ ID NO: 160) and QDGRMGF (SEQ ID NO: 161)), and mutant peptides thereof (e.g., mutation such as conservative substitutions of one, two, three, four or five amino acid residues), and peptides with one or more such amino acid sequences.

Preferably, the peptide capable of binding to biomolecule may be the peptide capable of binding to nucleic acid. Examples of the peptide capable of binding to nucleic acid include peptides disclosed in R. Tan et al., Proc. Natl. Acad. Sci. USA, 1995, vol. 92, p. 5282 (e.g., TRQARRN (SEQ ID NO: 162), TRQARRNRRRRWRERQR (SEQ ID NO: 163), TRRQRTRRARRNR (SEQ ID NO: 164), NAKTRRHERRRKLAIER (SEQ ID NO: 165), MDAQTRRRERRAEKQAQWKAA (SEQ ID NO: 166), and RKKRRQRRR (SEQ ID NO: 167)), peptides disclosed in R. Tan et al., Cell, 1993, vol. 73, p. 1031 (e.g., TRQARRNRRRRWRERQR (SEQ ID NO: 168)), peptides disclosed in Talanian et al., Biochemistry. 1992, vol. 31, p. 6871 (e.g., KRARNTEAARRSRARK (SEQ ID NO: 169)), and mutant peptides thereof (e.g., mutation such as conservative substitutions of one, two, three, four or five amino acid residues), and peptides with one or more such amino acid sequences.

Preferably, the peptide capable of binding to biological organic molecule may be the peptide capable of binding to saccharide. Examples of the peptide capable of binding to saccharide include peptides disclosed in K. Oldenburg et al., Proc. Natl. Acad. Sci. USA, 1992, vol. 89, No12, p. 5393-5397 (e.g., DVFYPYPYASGS (SEQ ID NO: 170) and RVWYPYGSYLTASGS (SEQ ID NO: 171)), peptides disclosed in K. Yamamoto et al., J. Biochem., 1992, vol. 111, p. 436 (e.g., DTWPNTEWS (SEQ ID NO: 172), DSYHNIW (SEQ ID NO: 173), DTYFGKAYNPW (SEQ ID NO: 174) and DTIGSPVNFW (SEQ ID NO: 175)), peptides disclosed in A. Baimiev et al., Mol. Biol. (Moscow), 2005, vol. 39, No. 1, p. 90 (e.g., TYCNPGWDPRDR (SEQ ID NO: 176) and TFYNEEWDLVIKDEH (SEQ ID NO: 177)), and mutant peptides thereof (e.g., mutation such as conservative substitutions of one, two, three, four or five amino acid residues), and peptides with one or more such amino acid sequences.

Preferably, the peptide capable of binding to biological organic molecule may be the peptide capable of binding to lipid. Examples of the peptide capable of binding to lipid include peptides disclosed in O. Kruse et al., Z. Naturforsch., 1995, vol. 50c, p. 380 (e.g., MTLILELVVI (SEQ ID NO: 178), MTSILEREQR (SEQ ID NO: 179) and MTTILQQRES (SEQ ID NO: 180)), peptides disclosed in O. Silva et al., Sci. Rep., 2016, Vol. 6, 27128 (e.g., VFQFLGKIIHHVGNFVHGFSHVF (SEQ ID NO: 181)), peptides disclosed in A. Filoteo et al., J. Biol. Chem., 1992, vol. 267 (17), p. 11800, (e.g., KKAVKVPKKEKSVLQGKLTRLAVQI (SEQ ID NO: 182)), and mutant peptides thereof (e.g., mutation such as conservative substitutions of one, two, three, four or five amino acid residues), and peptides with one or more such amino acid sequences.

Examples of the metal material include metals and metal compounds. Examples of the metals include titanium, gold, chromium, zinc, lead, manganese, calcium, copper, calcium, germanium, aluminum, gallium, cadmium, iron, cobalt, silver, platinum, palladium, hafnium, and tellurium. Examples of the metal compounds include oxides, sulfides, carbonates, arsenides, chlorides, fluorides, iodides and intermetallic compounds of such metals. Various peptides capable of binding to such metal materials have been reported (e.g., WO2005/010031; WO2012/086647; K. Sano et al., Langmuir, 2004, vol. 21, p. 3090; S. Brown, Nat. Biotechnol., 1997, Vol. 15, p. 269; K. Kjaergaard et al., Appl. Environ. Microbiol., 2000, vol. 66. p. 10; Umetsu et al., Adv. Mater., 17, 2571-2575 (2005); M. B. Dickerson et al., Chem. Commun., 2004, Vol. 15. p. 1776; C. E. Flynn et al., J. Mater. Chem., 2003, vol. 13. p. 2414). In the present invention, such various peptides can be used. It is also known that the peptide capable of binding to a metal can have a metal mineralization function while the peptide capable of binding to a metal compound can have a metal compound mineralization function (e.g., K. Sano et al., Langmuir, 2004, vol. 21, p. 3090; M. Umetsu et al., Adv. Mater., 2005, Vol. 17, p. 2571). As such, when the peptide capable of binding to the metal material is used as the peptide capable of binding to target material, the peptide capable of binding to the metal material has such a mineralization function.

Preferably, the peptide capable of binding to the metal material may refer to peptides capable of binding to titanium materials such as titanium or titanium compounds (e.g., titanium oxide), and peptides capable of binding to gold materials such as gold or gold compounds. Examples of the peptide capable of binding to the titanium material include peptides that are described in Examples and disclosed in WO2006/126595 (e.g., RKLPDA (SEQ ID NO: 7), peptides disclosed in M. J. Pender et al., Nano Lett., 2006, Vol. 6, No. 1, p. 40-44 (e.g., SSKKSGSYSGSKGSKRRIL (SEQ ID NO: 183)), peptides disclosed in I. Inoue et al., J. Biosci. Bioeng., 2006, vol. 122, No. 5, p. 528 (e.g., AYPQKFNNNFMS (SEQ ID NO: 184)), peptides disclosed in WO2006/126595 (e.g., RKLPDAPGMHTW (SEQ ID NO: 185) and RALPDA (SEQ ID NO: 186)), and mutant peptides thereof (e.g., mutation such as conservative substitutions of one, two, three, four or five amino acid residues), and peptides with one or more such amino acid sequences. Examples of the peptide capable of binding to the gold material include peptides that are described in Examples and disclosed in S. Brown, Nat. Biotechnol. 1997, vol. 15, p. 269 (e.g., MHGKTQATSGTIQS (SEQ ID NO: 21)), peptides disclosed in J. Kim et al., Acta Biomater., 2010, Vol. 6, No. 7, p. 2681 (e.g., TGTSVLIATPYV (SEQ ID NO: 187) and TGTSVLIATPGV (SEQ ID NO: 188)), peptides disclosed in K. Nam et. al., Science, 2006, vol. 312, No. 5775, p. 885 (e.g., LKAHLPPSRLPS (SEQ ID NO: 189)), and mutant peptides thereof (e.g., mutation such as conservative substitutions of one, two, three, four or five amino acid residues), and peptides with one or more such amino acid sequences.

Examples of the silicon material include silicon or silicon compounds. Examples of the silicon compound include silicon oxides (e.g., silicon monoxide (SiO), silicon dioxide (SiO₂)), silicon carbide (SiC), silane (SiH₄) and silicone rubber. Various peptides have been reported as peptides capable of binding to such a silicon material (For example, WO2006/126595; WO2006/126595; M. J. Pender et al., Nano Lett., 2006, vol. 6, No. 1, p. 40-44). Therefore, such a variety of peptides can be used in the present invention.

Preferably, the peptide capable of binding to the silicon material may be the peptide capable of binding to silicon or silicon compound (e.g., silicon oxide). Examples of such a peptide include peptides disclosed inWO2006/126595 (e.g., RKLPDA (SEQ ID NO: 7)), peptides disclosed in M. J. Pender et al., Nano Lett., 2006, vol. 6, No. 1, p. 40-44 (e.g., SSKKSGSYSGSKGSKRRIL (SEQ ID NO: 190)), peptides disclosed inWO2006/126595 (e.g., MSPHPHPRHHHT (SEQ ID NO: 191), TGRRRRLSCRLL (SEQ ID NO: 192) and KPSHHHHHTGAN (SEQ ID NO: 193)), and mutant peptides thereof (e.g., mutation such as conservative substitutions of one, two, three, four or five amino acid residues), and peptides with one or more such amino acid sequences.

Examples of the carbon material include carbon nanomaterials (e.g., carbon nanotube (CNT), carbon nanohorn (CNH)), fullerene (C60), graphene sheet and graphite. Various peptides have been reported as peptides capable of binding to such a carbon material (For example, Japanese Patent Application Laid-open No. 2004-121154; Japanese Patent Application Laid-open No. 2004-121154; and M. J. Pender et al., Nano Lett., 2006, vol. 6, No. 1, p. 40-44). Therefore, such a variety of peptides can be used in the present invention.

Preferably, the peptide capable of binding to the carbon material may be a peptide capable of binding to a carbon nanomaterial such as a carbon nanotube (CNT) or a carbon nanohorn (CNH). Examples of such peptides include peptides that are described below in Examples and disclosed in Japanese Patent Application Laid-open No. 2004-121154 (e.g., DYFSSPYYEQLF (SEQ ID NO: 194)), peptides disclosed in M. J. Pender et al., Nano Lett., 2006, vol. 6, No. 1, p. 40-44 (e.g., HSSYWYAFNNKT (SEQ ID NO: 195)), peptides disclosed in Japanese Patent Application Laid-open No. 2004-121154 (e.g., YDPFHII (SEQ ID NO: 196)), and mutant peptides thereof (e.g., mutation such as conservative substitutions of one, two, three, four or five amino acid residues), and peptides with one or more such amino acid sequences.

When a protease-degrading peptide is used as the functional peptide, examples of the protease include cysteine protease such as caspase and cathepsin (D. McIlwain1 et al., Cold Spring Harb Perspect Biol., 2013, vol. 5, a008656; V. Stoka et al., IUBMB Life. 2005, vol. 57, No. 4-5p. 347), collagenase (G. Lee et al., Eur J Pharm Biopharm., 2007, vol. 67, No. 3, p. 646), thrombin and Xa factor (R. Jenny et al., Protein Expr. Purif., 2003, vol. 31, p. 1; H. Xu et al., J. Virol., 2010, vol. 84, No. 2, p. 1076), and a virus-derived protease (C. Byrd et al., Drug Dev. Res., 2006, vol. 67, p. 501).

Examples of the protease-degrading peptide include peptides disclosed in E. Lee et al., Adv. Funct. Mater., 2015, vol. 25, p. 1279 (e.g., GRRGKGG (SEQ ID NO: 197)), G. Lee et al., Eur J Pharm Biopharm., 2007, vol. 67, No. 3, p. 646 (e.g., GPLGV (SEQ ID NO: 198) and GPLGVRG (SEQ ID NO: 199)), peptides disclosed in Y. Kang et al., Biomacromolecules, 2012, vol. 13, No. 12, p. 4057 (e.g., GGLVPRGSGAS (SEQ ID NO: 200)), peptides disclosed in R. Talanian et al., J. Biol. Chem., 1997, vol. 272, p. 9677 (e.g., YEVDGW (SEQ ID NO: 201), LEVDGW (SEQ ID NO: 202), VDQMDGW (SEQ ID NO: 203), VDVADGW (SEQ ID NO: 204), VQVDGW (SEQ ID NO: 205) and VDQVDGW (SEQ ID NO: 206)), peptides disclosed in Jenny et al., Protein Expr. Purif., 2003, vol. 31, p. 1 (e.g., ELSLSRLRDSA (SEQ ID NO: 207), ELSLSRLR (SEQ ID NO: 208), DNYTRLRK (SEQ ID NO: 209), YTRLRKQM (SEQ ID NO: 210), APSGRVSM (SEQ ID NO: 211), VSMIKNLQ (SEQ ID NO: 212), RIRPKLKW (SEQ ID NO: 213), NFFWKTFT (SEQ ID NO: 214), KMYPRGNH (SEQ ID NO: 215), QTYPRTNT (SEQ ID NO: 216), GVYARVTA (SEQ ID NO: 217), SGLSRIVN (SEQ ID NO: 218), NSRVA (SEQ ID NO: 219), QVRLG (SEQ ID NO: 220), MKSRNL (SEQ ID NO: 221), RCKPVN (SEQ ID NO: 222) and SSKYPN (SEQ ID NO: 223)), peptides disclosed in H. Xu et al., J. Virol., 2010, vol. 84, No. 2, p. 1076 (e.g., LVPRGS (SEQ ID NO: 224)), and mutant peptides thereof (e.g., mutation such as conservative substitutions of one, two, three, four or five amino acid residues), and peptides with one or more such amino acid sequences.

When a stabilizing peptide is used as the functional peptide, examples of the stabilizing peptide include peptide disclosed in X. Meng et al., Nanoscale, 2011, vol. 3, No. 3, p. 977 (e.g., CCALNN (SEQ ID NO: 225)), peptides disclosed in E. Falvo et al., Biomacromolecules, 2016, vol. 17, No. 2, p. 514 (e.g., PAS (SEQ ID NO: 226)), and mutant peptides thereof (e.g., mutation such as conservative substitutions of one, two, three, four or five amino acid residues), and peptides with one or more such amino acid sequences.

When a cell-permeable peptide is used as the functional peptide, examples of the cell-permeable peptide include peptides disclosed in Z. Guo et al., Biomed. Rep., 2016, vol. 4, No. 5, p. 528 (e.g., GRKKRRQRRRPPQ (SEQ ID NO: 227), RQIKIWFQNRRMKWKK (SEQ ID NO: 228), CGYGPKKKRKVGG (SEQ ID NO: 229), RRRRRRRR (SEQ ID NO: 230), KKKKKKKK (SEQ ID NO: 231), GLAFLGFLGAAGSTM (SEQ ID NO: 232), GAWSQPKKKRKV (SEQ ID NO: 233), LLIILRRRIRKQAHAHSK (SEQ ID NO: 234), MVRRFLVTL (SEQ ID NO: 235), RIRRACGPPRVRV (SEQ ID NO: 236), MVKSKIGSWILVLFV (SEQ ID NO: 237), SDVGLCKKRP (SEQ ID NO: 238), NAATATRGRSAASRPTQR (SEQ ID NO: 239), PRAPARSASRPRRPVQ (SEQ ID NO: 240), DPKGDPKGVTVT (SEQ ID NO: 241), VTVTVTGKGDPKPD (SEQ ID NO: 242), KLALKLALK (SEQ ID NO: 243), ALKAALKLA (SEQ ID NO: 244), GWTLNSAGYLLG (SEQ ID NO: 245), KINLKALAALAKKIL (SEQ ID NO: 246), RLSGMNEVLSFRW (SEQ ID NO: 247), SDLWEMMMVSLACQY (SEQ ID NO: 248) and PIEVCMYREP (SEQ ID NO: 249)), and mutant peptides thereof (e.g., mutation such as conservative substitutions of one, two, three, four or five amino acid residues), and peptides with one or more such amino acid sequences.

The functional peptide is preferably the peptide capable of binding to the target material. A preferred example of the peptide capable of binding to the target material is a peptide capable of binding to an organic substance. The peptide capable of binding to an organic substance is preferably the peptide capable of binding to biological organic molecule, and more preferably the peptide capable of binding to protein. Another example of the peptide capable of binding to the target material is a peptide capable of binding to an inorganic substance. The peptide capable of binding to the inorganic substance is preferably the peptide capable of binding to a metal material, and more preferably the peptide capable of binding to a titanium material or a gold material.

The fusion protein of the present invention may be modified at N-terminus region and/or C-terminus region thereof. The N-terminus of the ferritin monomer of animals such as the human ferritin monomer can be exposed on a surface of the multimer, while the C-terminus cannot be exposed on the surface. As such, the peptide part added to the N-terminus of the animal ferritin monomer cannot be exposed on the surface of the multimer for interacting with the target material existing outside the multimer, while the peptide part added to the C-terminus of the animal ferritin monomer is not exposed on the surface of the multimer and thus cannot interact with the target material existing outside the multimer (e.g.,WO2006/126595). However, it has been reported that the C-terminus of the animal ferritin monomer can be utilized in encapsulation of agents into an internal cavity of the multimer by modification of amino acid residues thereof (see, e.g., Y. J. Kang, Biomacromolecules. 2012, vol. 13(12), 4057). In the microbial ferritin monomer (i.e., Dps), both N-terminus and C-terminus can be exposed on the surface of the multimer. As such, both peptide parts added to the N-terminus and the C-terminus of the microbial ferritin monomer can be exposed on the surface of the multimer so as to interact with different target materials existing outside the multimer (e.g., WO2012/086647)

In a preferred embodiment, the fusion protein of the present invention may have a peptide part that is added to the N-terminus as the modification in the N-terminus region. An example of the peptide part to be added is the functional peptide described above. Other examples of the peptide part to be added include peptide components for improving solubility of the target protein (e.g., Nus-tag), peptide components acting as a chaperone (e.g., trigger factor), peptide components with other functions (e.g., a full-length protein or a part thereof) and linkers. As the peptide part to be added to the N-terminus of the fusion protein, a peptide that is the same as or different from the functional peptide to be inserted at the region between second and third α-helices can be used. From the viewpoint of achievement of the interaction with different target materials, different peptide is preferably used. Preferably, the peptide part to be added to the N-terminus of the fusion protein of the present invention is the functional peptide described above. The peptide part to be added to the N-terminus is preferably designed such that an amino acid residue (e.g., methionine residue) corresponding to a start codon is contained in the N-terminus. Such a design facilitates translation of the fusion protein of the present invention.

In another preferred embodiment, the modification at the C-terminus region of the fusion protein of the present invention may be carried out by substituting the amino acid residue at the C-terminus region with a reactive amino acid residue, inserting the reactive amino acid residue at the C-terminus region, or adding the reactive amino acid residue or a peptide containing the same (e.g., a peptide comprising 2 to 12, for example, or preferably 2 to 5 amino acid residues) to the C-terminus. Examples of such a C-terminus region include a region including 175th to 183rd (preferably 179th to 183rd) amino acid residues of the human ferritin H chain and 171st to 175th (preferably 173rd to 175th) amino acid residues of the human ferritin L chain. Such a modification allows the reactive amino acid residue to react with a certain substance (e.g., agent and target substance) and thus enables encapsulation of the certain substance into the internal cavity of the multimer through covalent bonds. Examples of such a reactive amino acid residue include cysteine residue having a thiol group, lysine residue having an amino group, arginine residue, asparagine residue, and glutamine residue, and cysteine residue is preferable. Preferably, the modification of the C-terminus region of the fusion protein of the present invention is the addition of the reactive amino acid residue or a peptide containing the same to the C-terminus.

The fusion protein of the present invention can be obtained by utilizing a host cell (host cell of the present invention) that contains polynucleotides encoding the fusion protein of the present invention and allows production of the fusion protein. Examples of the host cell used for producing the fusion protein of the present invention include cells derived from animals, insects, fishes, plants or microorganisms. The animals are preferably mammals or avian species (e.g., chicken), and more preferably mammals. Examples of the mammals include primates (e.g., humans, monkeys, and chimpanzees), rodents (e.g., mice, rats, hamsters, guinea pigs, and rabbits) and livestock and working mammals (e.g., cattle, pigs, sheep, goats, and horses).

In a preferred embodiment, the host cell is a human cell or a cell used for production of a human protein (e.g., Chinese hamster ovary (CHO) cells, and human fetal kidney-derived HEK 293 cells). When the human ferritin monomer and functional peptide are used as the fusion protein, such a host cell is preferably used from the viewpoint of clinical application to humans.

In another preferred embodiment, the host cell is a microorganism. From the viewpoint of mass production of the fusion protein and the like, such host cells may be used. Examples of the microorganism include bacteria and fungi. As the bacteria, any bacteria used as the host cells can be used. Examples of the bacterium include bacteria belonging to Bacillus (e.g., Bacillus subtilis), Corynebacterium (e.g., Corynebacterium glutamicum), Escherichia (e.g., Escherichia coli) and Pantoea (e.g., Pantoea ananatis). As the fungi, any fungi used as the host cells can be used. Examples of the fungus include fungi belonging to Saccharomyces (e.g., Saccharomyces cerevisiae) and Schizosaccharomyces (e.g., Schizosaccharomyces pombe). Alternatively, filamentous fungi may be used as the microorganism. Examples of the filamentous fungi include fungi belonging to Acremonium/Talaromyces, Trichoderma, Aspergillus, Neurospora, Fusarium, Chrysosporium, Humicola, Emericella and Hypocrea.

The host cell of the present invention preferably includes an expression unit containing a promoter operably linked to the polynucleotide in addition to the polynucleotide encoding the fusion protein of the present invention. The term "expression unit" refers to a unit that contains a certain polynucleotide to be expressed as a protein and the promoter operably linked to the polynucleotide and enables transcription of the polynucleotide and hence production of the protein encoded by the polynucleotide. The expression unit may further include an element such as a terminator, a ribosomal binding site, and a drug resistant gene. The expression unit may be DNA or RNA, and preferably DNA. The expression unit can be included in a genome region (e.g., a natural genome region that is a natural locus, in which a polynucleotide encoding the protein is inherently present, or an unnatural genome region that is not the natural locus), or a non-genomic region (e.g., a cytoplasm) in the microorganism (host cell). The expression units may be included at one or more (e.g., 1, 2, 3, 4 or 5) different positions in the genome region. Examples of specific forms of the expression unit included in the non-genomic region are plasmids, viral vectors, phages or artificial chromosomes.

The promoter constituting the expression unit is not particularly limited as long as it can express the protein encoded by the polynucleotide linked downstream thereof in the host cell. For example, the promoter may be homologous or heterologous to the host cell, but is preferably heterologous to the host cell. For example, a form or an inducible promoter that can be conventionally used for production of a recombinant protein can be used. As such a promoter, a promoter derived from a mammal, a promoter derived from a microorganism, a promoter derived from a virus or the like can be selected according to the type of the host cell to be used (e.g., a mammalian cell such as a human cell or a microorganism), as appropriate.

The host cell of the present invention can be prepared by any method known in the relevant field. For example, the host cell of the present invention can be prepared by a method using an expression vector (e.g., a competent cell method, an electroporation method, a calcium phosphate precipitation method), or a genome modification technique. When the expression vector is an integrative vector that causes homologous recombination with the genomic DNA of the host cell, the expression unit can be integrated to the genomic DNA of the host cell by transformation. When the expression vector is a non-integrative vector not causing homologous recombination with the genomic DNA of the host cell, the expression unit is not integrated to the genomic DNA of the host cell by transformation, and can be independently present as the expression vector from the genomic DNA in the host cell. According to genome editing techniques (e.g., CRISPR/Cas system, Transcription Activator-Like Effector Nucleases (TALEN), the expression unit can be integrated to the genomic DNA of the host cell and the expression unit that the host cell has inherently can be modified.

The expression vector may further include an element such as a terminator functioning in the host cell, a ribosome binding site, and a drug resistance gene in addition to the minimum unit described above as the expression unit. Examples of the drug resistance gene include a resistance gene resistant to drug such as tetracycline, ampicillin, kanamycin, hygromycin and phosphinothricin. The expression vector may also include a region that enables homologous recombination with the genome of the host cell for homologous recombination with the genomic DNA of the host cell. For example, the expression vector may be designed such that the expression unit contained therein is positioned between a pair of homologous regions (e.g., homology arms homologous to a specific sequence in the genome of the host cell, loxP and FRT). The genome region (the target of the homologous region) of the host cell to which the expression unit is introduced is not particularly limited, but may be a gene locus with a high level of expression in the host cell.

The expression vector may be a plasmid, a viral vector, a phage, or an artificial chromosome. The expression vector may also be an integrative vector or a non-integrative vector. The integrative vector may be a type of vector that is fully integrated to the genome of the host cell. Alternatively, the integrative vector may be a type of vector, a part (e.g., expression unit) of which is integrated to the genome of the host cell. The expression vector may be a DNA vector or an RNA vector (e.g., retrovirus). Such an expression vector can be selected according to the type of the used host cell (e.g., mammalian cells such as human cells or microorganisms), as appropriate.

A medium for culturing the host cell is known, and a suitable medium can be used corresponding to the kind of the host cell. A certain component (e.g., carbon source, nitrogen source, or vitamin) may be added to such a medium. The host cell is cultured generally at 16 to 42°C, preferably 25 to 37°C, generally for 5 to 168 hours, and preferably 8 to 72 hours. Examples of the culture method include a batch culture method, a fed-batch culture method and a continuous culture method. An inducer may be used to induce the expression of fusion protein.

The produced target protein can be purified or isolated from the host cell or the medium containing the host cell by a salting out method, a precipitation method (e.g., isoelectric point precipitation method and solvent precipitation method), a method utilizing molecular weight difference (e.g., dialysis, ultrafiltration, and gel filtration), a method utilizing specific affinity (e.g., affinity chromatography and ion exchange chromatography), a method utilizing difference in hydrophobicity (e.g., hydrophobic chromatography and reversed phase chromatography), or a combination thereof. When the fusion protein of the present invention is accumulated in the host cell, the fusion protein of the present invention can be obtained by disrupting (e.g., sonication and homogenization) or lysing (e.g., lysozyme treatment) the host cells and then treating the resulting disrupted product and lysate by the above method.

The present invention also provides the polynucleotide that encodes the fusion protein of the present invention as described above for the preparation of the fusion protein of the present invention, the expression vector containing the nucleotide, and the host cell.

The present invention also provides the multimer. The multimer comprises the fusion protein and can have the internal cavity. The detail of the fusion protein constituting the multimer of the present invention is described above. The multimer of the present invention can be produced autonomously by the expression of the fusion protein of the present invention. The number of monomer units constituting the multimer of the present invention can be determined depending on the origin of ferritin included in the fusion protein of the present invention. For example, when ferritin is derived from animals such as human, the multimer of the present invention is 24-mer. When ferritin is derived from a microorganism (e.g., Dps), the multimer of the present invention is 12-mer.

The multimer of the present invention may be a homomultimer comprising a single fusion protein as a monomer unit, or may be a heteromultimer comprising a plurality of different kinds (e.g., two kinds) of fusion proteins. For example, in animals such as human, it is known that most of ferritin exists as a heteromultimer comprising two kinds of subunits (H-chain and L-chain). Therefore, the heteromultimer can be used as the multimer of the present invention.

The multimer comprising a plurality of different types of fusion proteins can be obtained, for example, by producing different types of fusion proteins using the host cell containing a plurality of polynucleotides encoding different types of fusion proteins. The multimer can be obtained also by allowing a first monomer comprising a single fusion protein and a second monomer comprising a single fusion protein (different from the fusion protein constituting the first multimer) to coexist in the same medium (e.g., buffer solution) and letting them to stand. The monomer of the fusion protein can be prepared, for example, by letting the multimer of the present invention to stand in the buffer under a low pH (see, e.g., B. Zheng et al., Nanotechnology, 2010, vol. 21, p. 445602).

From the viewpoint of reducing the load of the preparation of the monomer constituting the multimer of the present invention (e.g., obtainment of the recombinant protein) and the like, the multimer of the present invention is preferably a homopolymer. The ferritin monomer part in the fusion protein constituting the homomultimer of the present invention described above is preferably either the animal ferritin monomer that is the animal ferritin H chain or the animal ferritin L chain, or the microorganism ferritin monomer (Dps monomer). The ferritin monomer part is more preferably either the human ferritin monomer that is human ferritin H chain or human ferritin L chain, or the Listeria innocua ferritin monomer (Dps monomer). The ferritin monomer part is still more preferably one selected from either one of the abovementioned (A1) to (C1) and one of the abovementioned (A2) to (C2), or one of the abovementioned (A3) to (C3). The functional peptide in the fusion protein constituting the homomultimer of the present invention is described above, and is preferably the peptide capable of binding to the target material. A preferred example of the peptide capable of binding to the target material is the peptide capable of binding to the organic substance. The peptide capable of binding to the organic substance is preferably the peptide capable of binding to the biological organic molecule, and more preferably the peptide capable of binding to the protein. Another preferred example of the peptide capable of binding to the target material is the peptide capable of binding to the inorganic substance. The peptide capable of binding to the inorganic substance is preferably the peptide capable of binding to the metal material, more preferably the peptide capable of binding to the titanium material or the gold material.

The fusion protein constituting the multimer of the present invention may be modified in its N-terminus region and/or C-terminus region. Preferably, in the fusion protein constituting the multimer of the present invention, the peptide part may be added to the N-terminus as the modification in the N-terminus region as described above. Examples of the peptide part to be added are described above. In the fusion protein constituting the multimer of the present invention, the modification in the C-terminus region described above may be carried out by substituting the amino acid residue in the C-terminus region with the reactive amino acid residue as described above, inserting the reactive amino acid residue in the C-terminus region, or adding the reactive amino acid residue or the peptide containing the same (the same as described above) to the C-terminus. Preferably, the modification of the C-terminus region of the fusion protein constituting the multimer of the present invention is the addition of the reactive amino acid residue or the peptide containing the same to the C-terminus.

The multimer of the present invention may contain a substance in the internal cavity through covalent bond(s) or non-covalent bond(s). For example, the encapsulation of the substance into the internal cavity of the multimer of the present invention through covalent bond(s) can be performed by modifying the C-terminus region of the fusion protein of the present invention as described above using the reactive amino acid residue. The encapsulation of the substance into the internal cavity of the multimer of the present invention through non-covalent bond(s) can be carried out by utilizing characteristics of ferritin capable of incorporating the substance (e.g., nanoparticle). A person skilled in the art can select the substance that can be encapsulated in the multimer of the present invention, as appropriate, by considering properties such as the size of the internal cavity of the multimer of the present invention and the charge of the amino acid residue in the region that can be involved in the incorporation of the substance in the multimer of the present invention (e.g., the region of C-terminus: see R. M. Kramer et al., 2004, J. Am. Chem. Soc., vol. 126, p. 13282). For example, human ferritin forms a cage-like structure having an internal cavity with an outer diameter of 12 nm (inner diameter of 7 nm). The microbial ferritin (Dps) forms a cage-like structure having an internal cavity having an outer diameter of 9 nm (inner diameter of 4.5 nm). The material to be encapsulated into such a multimer has such a size as to be encapsulated into such an internal cavity. It is reported that the encapsulation of the substance into the internal cavity of the multimer can be facilitated by changing the charge characteristics (e.g., the kinds and number of the amino acid residues with side chains capable of being positively or negatively charged) in the region that can be involved in the encapsulation of the substance in the multimer (see, e.g., R. M. Kramer et al., 2004, J. Am. Chem. Soc., vol. 126, p. 13282), and thus the multimer of the fusion protein having the region with the changed charge characteristics can be used in the present invention. Example of the substance that can be encapsulated in the multimer of the present invention through non-covalent bond(s) is the same inorganic material as the target material described above. Specific examples of the substance that can be encapsulated in the multimer of the present invention through non-covalent bond(s) include iron oxide, nickel, cobalt, manganese, phosphorus, uranium, beryllium, aluminum, cadmium sulfide, cadmium selenide, palladium, chromium, copper, silver, gadolinium complex, platinum cobalt, silicon oxide, cobalt oxide, indium oxide, platinum, gold, gold sulfide, zinc selenide and cadmium selenium. The encapsulation of the substance into the internal cavity of the multimer of the present invention through non-covalent bond(s) can be carried out by known methods, for example, in a similar way to the method of encapsulating the substance into the internal cavity of the multimer (see, e.g., I. Yamashita et al., Chem. Lett., 2005, vol. 33, p. 1158). Specifically, the substance can be encapsulated into the internal cavity of the multimer of the present invention by allowing the multimer of the present invention (or the fusion protein of the present invention) and the substance to be encapsulated to coexist in the buffer such as HEPES buffer, and then letting them to stand at an appropriate temperature (e.g., 0 to 37°C).

The multimer of the present invention may be provided as a set of different types of multimers incorporating a plurality of different types (e.g., two, three or four kinds) of substances when the substance is incorporated in the internal cavity. For example, when the multimer of the present invention is provided as a set of two kinds of multimers incorporating two kinds of substances, such a set can be obtained by combining a first multimer incorporating a first substance and a second multimer that is prepared separately from the first multimer and incorporates therein a second substance different from the first substance. With appropriate combinations of a variety of patterns of fusion proteins with that of encapsulated substances described above, a wide variety of multimers of the present invention can be obtained.

In a preferred embodiment, the multimer of the present invention is the multimer comprising the fusion protein that includes (a) the human ferritin monomer, and (b) the functional peptide inserted at the flexible linker region between α-helices in the B and C regions of the human ferritin monomer. The multimer has the internal cavity with the functional peptide capable of binding to the biological organic molecules. When the human ferritin monomer is used as the ferritin monomer in the fusion protein, the multimer can be the 24-mer. The multimer may contain a drug in the internal cavity. Such a multimer enables the encapsulation of drugs within the internal cavity as described above as well as bindings to the biological organic molecule that is the target of the functional peptide, and thus specifically deliver the drug to the biological target site in which the biological organic molecule is present. As such, the multimer of the present invention is useful, for example, as a drug delivery system (DDS). The multimer of the present invention achieves superior safety in clinical applications in view of the human ferritin monomer not exhibiting antigenicity and immunogenicity against human.

In another preferred embodiment, the multimer of the present invention is the multimer comprising the fusion protein that includes (a) the ferritin monomer, and (b) the functional peptide inserted at the flexible linker region between α-helices in the B and C regions of the ferritin monomer. The multimer has the internal cavity with the functional peptide capable of binding to the metal materials, silicon materials or carbon materials. The fusion protein may have the peptide part(s) capable of binding to the metal materials, silicon materials or carbon materials (preferably a material different from the material to be bound to the functional peptide) at the N-terminus and/or C-terminus. When the animal ferritin monomer is used as the ferritin monomer in a fusion protein, the multimer can be a 24-mer. When the microorganism ferritin monomer is used as the ferritin monomer in a fusion protein, the multimer can be a 12-mer. Such a multimer is useful for applications such as production of electronic devices (e.g., photoelectric conversion elements (e.g., solar cells such as dye-sensitized solar cells), hydrogen generating elements, water purifying materials, antibacterial materials, and semiconductor memory elements) and the like (e.g., WO2006/126595; WO2012/086647; K. Sano et al., Nano Lett., 2007, Vol. 7. p. 3200).

The present invention also provides a complex. The complex of the present invention contains the multimer of the present invention and the target material. In the complex of the present invention, the target material is bound to the functional peptide in the fusion protein constituting the multimer of the present invention. Preferred examples of the multimer of the present invention, the fusion protein constituting the multimer and the target material are as described above. The target material may be contained in another material or bound to another material. For example, cells containing biological organic molecules (e.g., cell surface antigen molecules) or a tissue containing such cells can be used as the target material. Furthermore, as the target material, a material fixed on a solid phase (e.g., plates such as well plates, supports, substrates, elements or devices) can be used.

In a preferred embodiment, the complex of the present invention is (1) the multimer comprising the fusion protein that includes (a) the human ferritin monomer, and (b) the functional peptide inserted at the flexible linker region between α-helices in the B and C regions of the human ferritin monomer. The multimer has the internal cavity with the functional peptide capable of binding to the biological organic molecules. The complex is (2) a complex that incorporates the biological organic molecule while the biological organic molecule is bound to the functional peptide. Such a complex is useful for researches and development of DDS (e.g., analysis of drug delivery system).

In another preferred example, the complex of the present invention is the multimer comprising the fusion protein that comprises (1) (a) the ferritin monomer, and (b) the functional peptide inserted at the flexible linker region between α-helices in the B and C regions of the ferritin monomer. The multimer has the internal cavity with the functional peptide capable of binding to the metal materials, silicon materials or carbon materials. The complex is (2) a complex that incorporates the metal material, the silicon material or the carbon material while the metal material, the silicon material or the carbon material is bound to the functional peptide. Such a complex is useful for applications such as production of electronic devices (e.g., photoelectric conversion elements (e.g., solar cells such as dye-sensitized solar cells), hydrogen generating elements, water purifying materials, antibacterial materials, and semiconductor memory elements) and the like (e.g., WO2006/126595; WO2012/086647; K. Sano et al., Nano Lett., 2007, Vol. 7. p. 3200).

### EXAMPLES

Hereinafter, the present invention is described in detail with reference to Examples, but the present invention is not limited to these Examples.

### <Example 1: Construction of multifunctional ferritin (1)>

Total synthesis was carried out for a DNA encoding a human-derived ferritin H chain (FTH-BC-TBP (SEQ ID NO: 8 and SEQ ID NO: 9)) in which a titanium recognizing peptide (minTBP1: RKLPDA (SEQ ID NO: 7)) was inserted for fusion at a flexible linker region between second and third α-helices counted from an N-terminus of a ferritin monomer comprising six α-helices.PCR was carried out using the synthesized DNA as a template as well as the following primers: 5'-GAAGGAGATATACATATGACGACCGCGTCCACCTCG-3' (SEQ ID NO: 10) and 5'-CTCGAATTCGGATCCTTAGCTTTCATTATCACTGTC-3' (SEQ ID NO: 11). PCR was carried out using pET20 (Merck KGaA) as a template as well as the following primers: 5'-TTTCATATGTATATCTCCTTCTTAAAGTTAAAC-3' (SEQ ID NO: 12) and 5'-TTTGGATCCGAATTCGAGCTCCGTCG-3' (SEQ ID NO: 13). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid (pET20-FTH-BC-TBP) carrying a gene encoding FTH-BC-TBP.

Total synthesis was carried out for a DNA encoding a human-derived ferritin H chain (FTH-D-TBP, SEQ ID NO: 250 and SEQ ID NO: 251) in which a titanium recognizing peptide (minTBP1) was inserted for fusion at a flexible linker region between fourth and fifth α-helices counted from the N-terminus of the ferritin monomer comprising six α-helices. The expression plasmid (pET20-FTH-D-TBP) carrying the gene encoding FTH-D-TBP was constructed using the synthesized DNA encoding FTH-D-TBP as a template as well as the same primers and reaction systems as those of FTH-BC-TBP.

Subsequently, Escherichia coli BL21 (DE3) into which the constructed pET20-FTH-BC-TBP was introduced was cultured in 100 mL of an LB medium (including 10 g/L of Bacto-tryptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into a HiPerp Q HP column (GE Healthcare Inc.) equilibrated with 50 mM Tris-HCl buffer (pH 8.0). Then, the target protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into a HiPrep 26/60 Sephacryl S-300 HR column (GE Healthcare Inc.) equilibrated with 10 mM Tris-HCl buffer (pH 8.0) to separate and purify FTH-BC-TBP by size. FTH-D-TBP was expressed using E.coli and purified in the same way.

The particle size and solution dispersibility of the obtained ferritin were evaluated by a dynamic light scattering method (DLS) using Zetasizer Nano ZS (Malvern Ltd.). As represented in FIGS. 1-1 and 1-2, both of FTH-BC-TBP and FTH-D-TBP exhibit mono-dispersion with an average diameter of about 12 nm, indicating formation of a high-order structure of a 24-mer without aggregation of the 24-mers.

### <Example 2: Activity evaluation of multifunctional ferritin (1)>

The adsorbabilities of two kinds of ferritin mutants, FTH-BC-TBP and FTH-D-TBP to a titanium film were evaluated by a quartz crystal microbalance (QCM) method.

First, 2 µL of piranha solution (a solution prepared by mixing concentrated sulfuric acid with hydrogen peroxide solution at 3:1) was put on a surface of a titanium film of a titanium film sensor cell (QCMSC-TI, Initium Inc.), and left to stand for 5 minutes, and then washed five times with 500 µL of water. The washing was repeated twice to remove organic substances on the surface of the titanium film. Subsequently, the titanium film sensor cell was set to AFFINIX QN µ (Initium Inc.), and 490 µL or 495 µL of 50 mM Tris-HCl buffer (pH 8.0) was put thereon. Next, an output value of the sensor was stabilized by stirring at a measurement temperature of 25°C, a rotation speed of 1,000 rpm, and leaving it to stand for about 30 minutes. For each measurement, 100 mg/L of ferritin mutant solution was added to the buffer placed on the titanium film sensor cell to control a final ferritin concentration to 1.9 nM in the solution. The concentration of the ferritin solution used for the evaluation was determined using a protein assay CBB solution (Nacalai Tesque, Inc.) with reference to bovine albumin as a standard. The following settings were used for the measurement to evaluate the adsorption amount on the surface of titanium film according to change in the frequency of QCM: molecular weight of the ferritin 24-mer of 529 kDa, reaction temperature of 25°C, stirring rotation speed of 1,000 rpm, frequency of 27 MHz and measurement interval of 5 seconds.

As a result, the change in the frequency of QCM could be confirmed by the addition of the buffer containing FTH-BC-TBP or FTH-D-TBP, demonstrating that the ferritin mutants exhibit adsorbability to the titanium film surface (FIG. 2).

Subsequently, the 100 mg/L of ferritin mutant solution was added to the buffer placed on the titanium film sensor cell to control the ferritin final concentration to 0.2 to 5.6 nM in the solution under the same conditions, for measurement of the frequency changes. Then, dissociation equilibrium constant KD values were determined by slopes that were obtained by plotting correlations between reciprocals of the different concentrations and reciprocals of the frequency changes.

As a result, the KD value of FTH-BC-TBP was 0.97 nM, as low as about one fourth of the KD value of FTH-D-TBP, 3.77 nM (FIG. 3). Analysis of covariance for the difference confirmed that there was a significant difference with a significance probability p value of 1% or less. This demonstrates that higher adsorbability to the target material was achieved by the ferritin with the titanium recognizing peptide inserted at the flexible linker region between second and third α-helices counted from the N-terminus of the ferritin monomer comprising six α-helices, compared to the ferritin with the peptide inserted between fourth and fifth α-helices.

### <Example 3: Construction of multifunctional ferritin (2)>

Total synthesis was carried out for a DNA encoding a human-derived ferritin H chain (FHBc (SEQ ID NO: 15 and SEQ ID NO: 16)) in which a cancer recognizing RGD peptide (ASDRGDFSG (SEQ ID NO: 14)) was inserted for fusion at the flexible linker region between second and third α-helices from the N-terminus of the ferritin monomer comprising six α-helices while cysteine is added to the C-terminus. PCR was carried out using the synthesized DNA as a template as well as the following primers: 5'-TTTCATATGACGACCGCGTCCACCTCG-3' (SEQ ID NO: 17) and 5'-TTTGGATCCTTAACAGCTTTCATTATCACTG-3' (SEQ ID NO: 18). PCR was carried out using pET20 (Merck KGaA) as a template as well as the following primers: 5'-TTTCATATGTATATCTCCTTCTTAAAGTTAAAC-3' (SEQ ID NO: 12) and 5'-TTTGGATCCGAATTCGAGCTCCGTCG-3' (SEQ ID NO: 13). The resulting PCR products were digested with restriction enzymes DpnI, BamHI and NdeI for ligation to construct an expression plasmid (pET20-FHBc) carryinga gene encoding FHBc.

Subsequently, Escherichia coli BL21 (DE3) into which the constructed pET20-FHBc was introduced was cultured in 100 mL of the LB medium (including 10 g/L of Bacto-tryptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into the HiPerp Q HP column (GE Healthcare Inc.) equilibrated with 50 mM Tris-HCl buffer (pH 8.0). The target protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into the HiPrep 26/60 Sephacryl S-300 HR column (GE Healthcare Inc.) equilibrated with 10 mM Tris-HCl buffer (pH 8.0) to separate and purify FHBc by size.

### <Example 4: Confirmation of high-order structure of multifunctional ferritin (1)>

A cage-like structure of the FHBc achieved by self-organization was confirmed by staining it with 3% phosphotungstic acid (PTA), and analyzing it under the transmission electron microscope (TEM) as illustrated in FIG. 4. Results show that FTBc has a diameter of 12 nm, which is the same size as that of naturally occurring human ferritin, confirming that the obtained FHBc enables the formation of cage-like structure without significantly losing a high-order structure of protein even when the peptide is inserted at the flexible linker region between second and third α-helices.

A subsequent experiment was carried out in an attempt to form iron oxide nanoparticles inside internal cavities of the ferritins for confirming that FHBc has a function as ferritin while maintaining the internal cavity.

A 10 mL FTBc-containing Tris-HCl buffer (including 50 mM Tris-HCl (pH 8.5), 0.5 mg/mL of FTBc, 300 mM NaCl and 1 mM ammonium sulfate iron at final concentrations) was prepared and left to stand at 4°C for 30 minutes, resulting in a change in the color of the solution to orange. It suggested that the iron oxide nanoparticles were formed inside the internal cavity of ferritin. After cooled and left to stand, the resulting solution was centrifuged at 6,500 rpm for 15 minutes. After collection of the supernatant, the resulting solvent was replaced with 10 mM Tris-HCl buffer (pH 8.0) by centrifugal ultrafiltration using Vivaspin 20-100K (GE Healthcare Inc.). The resulting solution was injected into the HiPrep 16/60 Sephacryl S-300 HR column (GE Healthcare Inc.) equilibrated with 10 mM Tris-HCl buffer (pH 8.0) to separate and purify FHBc with encapsulated iron nanoparticles.

The particle size and solution dispersibility of the obtained ferritin with encapsulated iron nanoparticles were evaluated by the dynamic light scattering method (DLS) using Zetasizer Nano ZS (Malvern Ltd.). As represented in FIG. 5, FHBc with encapsulated iron nanoparticles is confirmed to exhibit mono-dispersion with an average diameter of 16 nm or less, indicating no aggregation of FHBc.

### <Example 5: Construction of multifunctional ferritin (3)>

Total synthesis was carried out for a DNA encoding the human-derived ferritin H chain (FTH-BC-GBP (SEQ ID NO: 20 and SEQ ID NO: 21)) in which a gold recognizing peptide (GBP1: MHGKTQATSGTIQS (SEQ ID NO: 19)) was inserted for fusion at the flexible linker region between second and third α-helices counted from the N-terminus of the ferritin monomer comprising six α-helices.PCR was carried out using the synthesized DNA as a template as well as the following primers: 5'-GAAGGAGATATACATATGACGACCGCGTCCACCTCG-3' (SEQ ID NO: 10) and 5'-CTCGAATTCGGATCCTTAGCTTTCATTATCACTGTC-3' (SEQ ID NO: 11). PCR was carried out using pET20 (Merck KGaA) as a template as well as the following primers: 5'-TTTCATATGTATATCTCCTTCTTAAAGTTAAAC-3' (SEQ ID NO: 12) and 5'-TTTGGATCCGAATTCGAGCTCCGTCG-3' (SEQ ID NO: 13). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid carrying the synthesized gene. In the confirmed nucleic acid sequence of the synthesized gene carried on this plasmid, methionine was deleted at the beginning of amino acid sequence of the gold recognizing peptide GBP1. For modifying the deletion of methionine, PCR was carried out using the constructed plasmid as a template DNA as well as primers with the following sequences: 5'-ATGCATGGCAAAACCCAGGCGACCAG-3' (SEQ ID NO: 22) and 5'-ACCCTTGATATCCTGAAGGA-3' (SEQ ID NO: 23). The resulting PCR products were purified using Wizard DNA Clean-Up System (Promega Corporation), then treated with T4 Polynucleotide Kinase (Takara Bio Inc.) and left to stand at 37°C for 30 minutes for the purpose of phosphorylating the 5' terminus of the PCR product. The resulting DNA was subjected to self-ligation to construct an expression plasmid (pET20-FTH-BC-GBP) carrying FTH-BC-GBP.

Total synthesis was carried out also for a DNA encoding a human-derived ferritin H chain (FTH-D-GBP, SEQ ID NO: 252 and SEQ ID NO: 253) in which a gold recognizing peptide (GBP1) was inserted for fusion between fourth and fifth α-helices counted from the N-terminus of the ferritin monomer comprising six α-helices. The expression plasmid (pET20-FTH-D-GBP) carrying the gene encoding FTH-D-GBP was also constructed using the synthesized DNA encoding FTH-D-GBP as a template as well as the same primers and reaction systems as those of FTH-BC-GBP. Since methionine was deleted at the beginning of the amino acid sequence of the gold recognizing peptide GBP1, an expression plasmid (pET20-FTH-D-GBP) carrying FTH-D-GBP was constructed by carrying out PCR with primers of 5'-ATGCATGGCAAAACCCAGGCGACCAG-3' (SEQ ID NO: 22) and 5'-ATGTGTCTCAATGAAGTCACACAA-3' (SEQ ID NO: 254) and then treating it with T4 Polynucleotide Kinase in the same way as in the case of FTH-BC-GBP.

Subsequently, Escherichia coli BL21 (DE3) into which the constructed pET20-FTH-BC-GBP was introduced was cultured in 100 mL of the LB medium (including 10 g/L of Bacto-tryptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into the HiPerp Q HP column (GE Healthcare Inc.) equilibrated with 50 mM Tris-HCl buffer (pH 8.0). Then, the target protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into the HiPrep 26/60 Sephacryl S-300 HR column (GE Healthcare Inc.) equilibrated with 10 mM Tris-HCl buffer (pH 8.0) to separate and purify FTH-BC-GBP by size. FTH-D-GBP was expressed using E.coli and purified in the same way.

The particle size and solution dispersibility of the obtained ferritin were evaluated by the dynamic light scattering method (DLS) using Zetasizer Nano ZS (Malvern Ltd.). As represented in FIGS. 6-1 and 6-2, FTH-BC-GBP and FTH-D-GBP were confirmed to exhibit mono-dispersion with an average diameter of about 12 nm and formation of high-order structure of 24-mer, indicating no aggregation between the 24-mers.

### <Example 6: Activity evaluation of multifunctional ferritin (2)>

The adsorbabilities of two kinds of ferritin mutants, FTH-BC-GBP and FTH-D-GBP to a gold film were evaluated by the quartz crystal microbalance (QCM) method.

First, 2 µL of piranha solution (the solution prepared by mixing concentrated sulfuric acid with hydrogen peroxide solution at 3:1) was put on a surface of a gold film of a gold film sensor cell (QCMSC-AU, Initium Inc.), and left to stand for 5 minutes, and then washed five times with 500 µL of water. The washing was repeated twice to remove organic substances on the surface of the gold film. Subsequently, the gold film sensor cell was set to AFFINIX QN µ (Initium Inc.), and 490 µL or 495 µL of 50 mM phosphate buffer (pH 6.0) was put thereon. Next, an output value of the sensor was stabilized by stirring at a measurement temperature of 25°C, a rotation speed of 1,000 rpm, and leaving it to stand for about 30 minutes. The 100 mg/L of ferritin mutant solution was added to the buffer placed on the gold film sensor cell to control a final ferritin concentration to 0.3 to 5.4 nM in the solution, for the measurement of frequency changes. The concentration of the ferritin solution used for the evaluation was determined using the protein assay CBB solution (Nacalai Tesque, Inc.) with reference to bovine albumin as a standard. The following settings were used for the measurement to evaluate the adsorption amount on the surface of gold film according to the frequency change: molecular weight of the ferritin 24-mer of 546 kDa, QCM frequency of 27 MHz and measurement interval of 5 seconds. Then, the dissociation equilibrium constant KD values were determined by the slopes that were obtained by plotting correlations between reciprocals of the different concentrations and reciprocals of the frequency changes.

As a result, the KD value of FTH-BC-GBP was 0.42 nM, as low as about one seventh of the KD value of FTH-D-GBP, 3.10 nM (FIG. 7). Analysis of covariance for the difference confirmed that there was a significant difference with a significance probability p value of 1% or less. This demonstrates that higher adsorbability to the target material was achieved by the ferritin with the gold recognizing peptide inserted at the flexible linker region between second and third α-helices counted from the N-terminus of the H chain ferritin monomer comprising six α-helices, compared to the ferritin with the peptide inserted between fourth and fifth α-helices

### <Example 7: Construction of multifunctional ferritin (4)>

Total synthesis was carried out for a DNA encoding a human-derived ferritin L chain (FTL-BC-GBP (SEQ ID NO: 24 and SEQ ID NO: 25), FIG. 8) in which the gold recognizing peptide (GBP1: MHGKTQATSGTIQS (SEQ ID NO: 19)) was inserted for fusion at a flexible linker region between second and third α-helices counted from an N-terminus of the ferritin monomer comprising six α-helices. PCR was carried out using the synthesized DNA as a template as well as the following primers: 5'-GAAGGAGATATACATATGAGCTCCCAGATTCGTCAG-3' (SEQ ID NO: 26) and 5'-CTCGAATTCGGATCCTTAGTCGTGCTTGAGAGTGAG-3' (SEQ ID NO: 27). PCR was carried out using pET20 (Merck KGaA) as a template as well as the following primers: 5'-TTTCATATGTATATCTCCTTCTTAAAGTTAAAC-3' (SEQ ID NO: 12) and 5'-TTTGGATCCGAATTCGAGCTCCGTCG-3' (SEQ ID NO: 13). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid carrying the synthesized gene. In the confirmed nucleic acid sequence of the synthesized gene loaded on this plasmid, methionine was deleted at the beginning of amino acid sequence of the gold recognizing peptide GBP1. For modifying the deletion of methionine, PCR was carried out using the constructed plasmid as a template DNA as well as the following primers: 5'-ATGCATGGCAAAACCCAGGCGACCAG-3' (SEQ ID NO: 22) and 5'-ACCCTTGATGTCCTGGAAGAGA-3' (SEQ ID NO: 28). The resulting PCR products were purified using Wizard DNA Clean-Up System (Promega Corporation), then treated with T4 Polynucleotide Kinase (Takara Bio Inc.) and left to stand at 37°C for 30 minutes for the purpose of phosphorylating the 5' terminus of the PCR product. The resulting DNA was subjected to self-ligation to construct an expression plasmid (pET20-FTL-BC-GBP) carrying FTL-BC-GBP.

Total synthesis was carried out also for a DNA encoding a human-derived ferritin L chain (FTL-DE-GBP (SEQ ID NO: 29 and SEQ ID NO: 30) FIG. 9) in which the gold recognizing peptide (GBP1) was inserted for fusion at the flexible linker region between fifth and sixth α-helices counted from the N-terminus of the ferritin monomer comprising six α-helices. The expression plasmid (pET20-FTH-DE-GBP) carrying the gene encoding FTL-DE-GBP was also constructed using the synthesized DNA encoding FTL-DE-GBP as a template as well as the same primers and reaction systems as those of FTL-BC-GBP. Since methionine was deleted at the beginning of the amino acid sequence of the gold recognizing peptide GBP1, an expression plasmid (pET20-FTL-DE-GBP) carrying FTL-DE-GBP was constructed by carrying out PCR with primers of 5'-ATGCATGGCAAAACCCAGGCGACCAG-3' (SEQ ID NO: 22) and 5'-CATACCCAGCCTGTGGAGGT-3' (SEQ ID NO: 31) and then treating it with T4 Polynucleotide Kinase in the same way as in the case of FTL-BC-GBP.

Subsequently, Escherichia coli BL21 (DE3) into which the constructed pET20-FTL-BC-GBP was introduced was cultured in 100 mL of the LB medium (including 10 g/L of Bacto-tryptone, 5 g/L of Bacto-yeast extract, 5 g/L NaCl and 100 mg/L of ampicillin) at 30°C for 24 hours using flasks. The resulting bacterial cells were sonicated for cell disruption, and then the resulting supernatant was heated at 60°C for 20 minutes. The supernatant obtained after the heating was injected into the HiPerp Q HP column (GE Healthcare Inc.) equilibrated with 50 mM Tris-HCl buffer (pH 8.0). Then, the target protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into the HiPrep 26/60 Sephacryl S-300 HR column (GE Healthcare Inc.) equilibrated with 10 mM Tris-HCl buffer (pH 8.0) to separate and purify FTL-BC-GBP by size. FTL-DE-GBP was expressed using E.coli and purified in the same way.

### <Example 8: Activity evaluation of multifunctional ferritin (3)>

The adsorbabilities of two kinds of ferritin mutants, FTL-BC-GBP and FTL-DE-GBP to the gold film were evaluated by the quartz crystal microbalance (QCM) method.

First, 2 µL of piranha solution (the solution prepared by mixing concentrated sulfuric acid with hydrogen peroxide solution at 3:1) was put on the surface of the gold film of the gold film sensor cell (QCMSC-AU, Initium Inc.), and left to stand for 5 minutes, and then washed five times with 500 µL of water. The washing was repeated twice to remove organic substances on the surface of the gold film. Subsequently, the gold film sensor cell was set to AFFINIX QN µ (Initium Inc.), and 490 µL or 495 µL of 50 mM phosphate buffer (pH 6.0) was put thereon. Next, the output value of the sensor was stabilized by stirring at a measurement temperature of 25°C, a rotation speed of 1,000 rpm, and leaving it to stand for about 30 minutes. The 100 mg/L of ferritin mutant solution was added to the buffer placed on the gold film sensor cell to control a final ferritin concentration to 0.2 to 4.9 nM in the solution, for the measurement of frequency changes. The concentration of the ferritin solution used for the evaluation was determined using the protein assay CBB solution (Nacalai Tesque, Inc.) with reference to bovine albumin as a standard. The following settings were used for the measurement to evaluate the adsorption amount on the surface of gold film according to the frequency change: molecular weight of the ferritin 24-mer of 518 kDa, QCM frequency of 27 MHz and measurement interval of 5 seconds. Then, the dissociation equilibrium constant KD values were determined by the slopes that were obtained by plotting correlations between reciprocals of the different concentrations and reciprocals of the frequency changes.

As a result, the KD value of FTL-BC-GBP was 1.15 nM, as low as about 70% of the KD value of FTL-DE-GBP, 1.68 nM (FIG. 10). Analysis of covariance for the difference confirmed that there was a significant difference with a significance probability p value of 5% or less. This demonstrates that higher adsorbability to the target material was achieved by the ferritin with the gold recognizing peptide inserted at the flexible linker region between second and third α-helices counted from the N-terminus of the L chain ferritin monomer comprising six α-helices, compared to the ferritin with the peptide inserted at the flexible linker region between fifth and sixth α-helices.

The aforementioned results confirm that the peptides inserted at the flexible linker region between second and third α-helices counted from the N-terminus are highly effective in both of H and L chains of human ferritin.

### <Example 9: Construction of multifunctional microorganism-derived ferritin (Dps)>

Dps, a homolog protein of ferritin in microorganisms, has twelve monomers each having a structure analogous to that of ferritin. The twelve monomers are bound together to form a cage having an outer diameter of 9 nm and an inner diameter of 4.5 nm smaller than the ferritin. The three-dimensional structures of the monomers of ferritin and Dps are very similar to each other. It is known that a small α-helix comprising 7 amino acids is formed in a flexible linker region of Dps that corresponds to the flexible linker region between the second and third α-helices counted from the N-terminus of the ferritin monomer comprising six α-helices (Int. J. Mol. Sci. 2011; 12 (8): 5406-5421). A Listeria innocua-derived Dps (BCDps-CS4, SEQ ID NO: 33 and SEQ ID NO: 34) with a heterologous peptide (QVNGLGERSQQM (SEQ ID NO: 32)) being inserted at a C-terminus and a region corresponding to that of ferritin, was constructed.

First, total synthesis was carried out for a part of BCDps-CS4 gene. PCR was carried out using the synthesized gene as a template as well as the following primers: 5'-TTTCATATGAAAACAATCAACTCAGTAG-3' (SEQ ID NO: 35) and 5'-TTTGGATCCTTACATCTGCTGACTCCGCTCACCCAAACCATTCACCTGTTCTAATGGAG CTTTTCCAAG-3' (SEQ ID NO: 36). PCR was carried out using pET20 (Merck KGaA) as a template as well as the following primers: 5'- TTTCATATGTATATCTCCTTCTTAAAGTTAAAC-3' (SEQ ID NO: 12) and 5'-TTTGGATCCGAATTCGAGCTCCGTCG-3' (SEQ ID NO: 13). The resulting PCR products were digested with restriction enzymes DpnI, BamHI, NdeI for ligation to construct an expression plasmid (pET20-BCDps-CS4) carrying the gene encoding BCDps-CS4.

Subsequently, Escherichia coli BL21 (DE3) into which the constructed pET20-BCDps-CS4 was introduced was cultured in 100 mL of the LB medium (including 10 g/L of Bacto-tryptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into the HiPerp Q HP column (GE Healthcare Inc.) equilibrated with 50 mM Tris-HCl buffer (pH 8.0). Then, the target protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into the HiPrep 26/60 Sephacryl S-300 HR column (GE Healthcare Inc.) equilibrated with 10 mM Tris-HCl buffer (pH 8.0) to separate and purify BCDps-CS4 by size.

### <Example 10: Confirmation of high-order structure of multifunctional Dps>

A cage-like structure of the obtained BCDps-CS4 achieved by self-organization was confirmed by staining it with 3% phosphotungstic acid, and analyzing it under the transmission electron microscope (TEM) as illustrated in FIG. 11. Results show that BCDps-CS4 has a diameter of 9 nm, which is the same size as that of naturally occurring Dps, confirming that the Dps enables the formation of the same cage-like structure as that of naturally occurring Dps without significantly losing a high-order structure of protein even when the peptide is inserted at the flexible linker region between second and third α-helices of human ferritin.

### <Example 11: Construction of multifunctional ferritin (5)>

Total synthesis was carried out for a DNA encoding a human-derived ferritin H chain (FTH-DE-GBP (SEQ ID NO: 255 and SEQ ID NO: 256)) in which the gold recognizing peptide (GBP1: MHGKTQATSGTIQS (SEQ ID NO: 19)) was inserted for fusion at the flexible linker region between fifth and sixth α-helices counted from the N-terminus of the ferritin monomer comprising six α-helices. PCR was carried out using the synthesized DNA as a template as well as the following primers: 5'-GAAGGAGATATACATATGACGACCGCGTCCACCTCG-3' (SEQ ID NO: 10) and 5'-CTCGAATTCGGATCCTTAGCTTTCATTATCACTGTC-3' (SEQ ID NO: 11). PCR was carried out using pET20 (Merck KGaA) as a template as well as the following primers: 5'-TTTCATATGTATATCTCCTTCTTAAAGTTAAAC-3' (SEQ ID NO: 12) and 5'-TTTGGATCCGAATTCGAGCTCCGTCG-3' (SEQ ID NO: 13). The resulting PCR products were purified using Wizard DNA Clean-Up System (Promega Corporation), and then subjected to In-Fusion enzyme treatment at 50°C for 15 minutes using In-Fusion HD Cloning Kit (Takara Bio Inc.) to construct an expression plasmid (pET20-FTH-DE-GBP) carrying FTH-DE-GBP for the multifunctional ferritin construction was loaded.

Subsequently, Escherichia coli BL21 (DE3) into which the constructed pET20-FTH-DE-GBP was introduced was cultured in 100 mL of the LB medium (including 10 g/L of Bacto-tryptone, 5 g/L of Bacto-yeast extract, 5 g/L NaCl and 100 mg/L of ampicillin) at 37°C for 24 hours using flasks. The resulting bacterial cells were sonicated for cell disruption, and then the resulting supernatant was heated at 60°C for 20 minutes. The supernatant obtained after the heating was injected into the HiPerp Q HP column (GE Healthcare Inc.) equilibrated with 50 mM Tris-HCl buffer (pH 8.0). Then, the target protein was separated and purified by applying a concentration gradient of the salt from 0 mM to 500 mM NaCl in 50 mM Tris-HCl buffer (pH 8.0). The solvent of the solution containing the protein was replaced with 10 mM Tris-HCl buffer (pH 8.0) by centrifugal ultrafiltration using Vivaspin 20-100K (GE Healthcare Inc.). The resulting solution was injected into the HiPrep 26/60 Sephacryl S-300 HR column (GE Healthcare Inc.) equilibrated with 10 mM Tris-HCl buffer (pH 8.0) to separate and purify FTH-DE-GBP by size.

### <Example 12: Activity evaluation of multifunctional ferritin (4)>

The adsorbabilities of two kinds of ferritin mutants, FTH-BC-GBP and FTH-DE-GBP to the gold film were evaluated by the quartz crystal microbalance (QCM) method.

First, 2 µL of piranha solution (the solution prepared by mixing concentrated sulfuric acid with hydrogen peroxide solution at 3:1) was put on the surface of the gold film of the gold film sensor cell (QCMSC-AU, Initium Inc.), and left to stand for 5 minutes, and then washed five times with 500 µL of water. The washing was repeated twice to remove organic substances on the surface of the gold film. Subsequently, the gold film sensor cell was set to AFFINIX QN µ (Initium Inc.), and 490 µL or 495 µL of 50 mM phosphate buffer (pH 6.0) was put thereon. Next, the output value of the sensor was stabilized by stirring at a measurement temperature of 25°C, a rotation speed of 1,000 rpm, and leaving it to stand for about 30 minutes. The 100 mg/L of ferritin mutant solution was added to the buffer placed on the gold film sensor cell to control a final ferritin concentration to 0.2 to 2.6 nM in the solution, for the measurement of frequency changes. The concentration of the ferritin solution used for the evaluation was determined using the protein assay CBB solution (Nacalai Tesque, Inc.) with reference to bovine albumin as a standard. The following settings were used for the measurement to evaluate the adsorption amount on the surface of gold film according to the frequency change: molecular weight of the ferritin 24-mer of 546 kDa, QCM frequency of 27 MHz and measurement interval of 5 seconds. Then, the dissociation equilibrium constant KD values were determined from the slopes that were obtained by plotting correlations between reciprocals of the different concentrations and reciprocals of the frequency changes.

As a result, the KD value of FTH-DE-GBP was 1.90 nM, as low as about one fifth of the KD value of FTH-BC-GBP measured in Example 6, 0.42 nM (FIG. 12). Analysis of covariance for the difference confirmed that there was a significant difference with a significance probability p value of 5% or less. This demonstrates that higher adsorbability to the target material was achieved by the ferritin with the gold recognizing peptide inserted at the flexible linker region between second and third α-helices counted from the N-terminus of the H chain ferritin monomer comprising six α-helices, compared to the ferritin with the peptide inserted between fifth and sixth α-helices.

### INDUSTRIAL APPLICABILITY

The multimer of the present invention is promising for applications such as new drug delivery systems (DDS) and preparation of electronic devices. For example, when the ferritin monomer in the fusion protein constituting the multimer of the present invention is the human ferritin monomer, the multimer of the present invention is useful as DDS. The multimer of the present invention achieves superior safety in clinical applications in view of the human ferritin monomer not exhibiting antigenicity or immunogenicity against human. When the ferritin monomer is the microorganism ferritin monomer, the multimer of the present invention is useful for the preparation of electronic devices.

The fusion protein of the present invention is useful, for example, for preparation of the multimer of the present invention.

The complex of the present invention is useful, for example, for applications such as research and development of new drug delivery systems (DDS) and preparation of electronic devices.

The polynucleotides, expression vectors and host cells of the present invention facilitate the preparation of the fusion protein of the present invention. Accordingly, the polynucleotides, expression vectors and host cells of the present invention are useful, for example, for the preparation of the multimer of the present invention.

## Claims

1. A fusion protein comprising (a) a ferritin monomer, and (b) a functional peptide inserted at a flexible linker region between α-helices in a B region and a C region of the ferritin monomer.

2. The fusion protein according to claim 1, wherein the ferritin monomer is a human ferritin monomer.

3. The fusion protein according to claim 1 or 2, wherein the human ferritin monomer is a human ferritin H chain.

4. The fusion protein according to claim 1 or 2, wherein the human ferritin monomer is a human ferritin L chain.

5. The fusion protein according to claim 1, wherein the ferritin monomer is a Dps monomer.

6. The fusion protein according to any one of claims 1 to 5, wherein the functional peptide is a peptide capable of binding to a target material.

7. The fusion protein according to claim 6, wherein the target material is an inorganic substance.

8. The fusion protein according to claim 7, wherein the inorganic substance is a metal material.

9. The fusion protein according to claim 6, wherein the target material is an organic substance.

10. The fusion protein according to claim 9, wherein the organic substance is a biological organic molecule.

11. The fusion protein according to claim 10, wherein the biological organic molecule is a protein.

12. The fusion protein according to any one of claims 1 to 11, wherein a cysteine residue or a peptide containing the cysteine residue is added to a C-terminus of the fusion protein.

13. A multimer comprising a fusion protein and has an internal cavity, the fusion protein comprising (a) a ferritin monomer, and (b) a functional peptide inserted at a flexible linker region between α-helices in a B region and a C region of the ferritin monomer.

14. A complex comprising (1) the multimer according to claim 13, and (2) a target material,
wherein the target material is bound to the functional peptide in the fusion protein.

15. A polynucleotide encoding the fusion protein according to any one of claims 1 to 12.

16. An expression vector comprising the polynucleotide according to claim 15.

17. A host cell comprising the polynucleotide according to claim 15.
